(19) Europäisches Patentamt
European Patent Office
Office européen des brevets

(11) Publication number: **0 678 508 A1**

(12) **EUROPEAN PATENT APPLICATION**

(21) Application number: **95201691.3**

(22) Date of filing: **29.08.92**

(51) Int. Cl.[6]: **C07D 209/42**, C07D 209/30, C07D 401/12, C07D 405/12, C07D 403/12, C07D 401/06, C07D 405/06, C07D 413/06, A61K 31/40, A61K 31/44, A61K 31/41

This application was filed on 22 - 06 - 1995 as a divisional application to the application mentioned under INID code 60.

(30) Priority: **06.09.91 US 756013**
**07.02.92 US 832260**
**09.04.92 US 866765**

(43) Date of publication of application:
**25.10.95 Bulletin 95/43**

(60) Publication number of the earlier application in accordance with Art.76 EPC: **0 530 907**

(84) Designated Contracting States:
**AT BE CH DE DK ES FR GB GR IE IT LI LU NL PT SE**

(71) Applicant: **MERCK & CO. INC.**
**126, East Lincoln Avenue**
**P.O. Box 2000**
**Rahway**
**New Jersey 07065-0900 (US)**

(72) Inventor: **Williams, Theresa M.**
**260 Shadynook Road**
**Harleysville, PA 19438 (US)**
Inventor: **Ciccarone, Terrence M.**
**RD1, Box 516, Township Woods Road**
**East Greenville, PA 18041 (US)**
Inventor: **Saari, Walfred S.**
**1740 Wagon Wheel Lane**
**Lansdale, PA19446 (US)**
Inventor: **Wai, John S.**
**208 Tanglewood Way**
**Harleysville, PA 19438 (US)**
Inventor: **Greenlee, William J.**
**115 Herrick Avenue**
**teaneck, NJ 07666 (US)**
Inventor: **Balani, Suresh K.**
**2813 Denbeigh Drive**
**Hatfield, PA 19440 (US)**
Inventor: **Goldman, Mark E.**
**1510 Bridal Path**
**Lansdale, PA 19446 (US)**
Inventor: **Theoharides, Anthony D.**
**417 Shipwrighter Way**
**Lansdale, PA 19446 (US)**

(74) Representative: **Cole, William Gwyn et al**
**European Patent Department**
**Merck & Co., Inc.**
**Terlings Park**
**Eastwick Road**
**Harlow**
**Essex CM20 2OR (GB)**

(54) **Indoles as inhibitors of HIV reverse transcriptase.**

(57) Novel indole compounds inhibit HIV reverse transcriptase, and are useful in the prevention or treatment of infection by HIV and the treatment of AIDS, either as compounds, pharmaceutically acceptable salts, pharmaceutical composition ingredients, whether or not in combination with other antivirals, anti-infectives, immunomodulators, antibiotics or vaccines. Methods of treating AIDS and methods of preventing or treating infection by HIV are also described.

EP 0 678 508 A1

The present invention is concerned with compounds which inhibit the reverse transcriptase encoded by human immunodeficiency virus (HIV) or pharmaceutically acceptable salts thereof and are of value in the prevention of infection by HIV, the treatment of infection by HIV and the treatment of the resulting acquired immune deficiency syndrome (AIDS). It also relates to pharmaceutical compositions containing the compounds and to a method of use of the present compounds and other agents for the treatment of AIDS and viral infection by HIV.

## BACKGROUND OF THE INVENTION

A retrovirus designated human immunodeficiency virus (HIV) is the etiological agent of the complex disease that includes progressive destruction of the immune system (acquired immune deficiency syndrome; AIDS) and degeneration of the central and peripheral nervous system. This virus was previously known as LAV, HTLV-III, or ARV. A common feature of retrovirus replication is reverse transcription of the RNA genome by a vi rally encoded reverse transcriptase to generate DNA copies of HIV sequences, a required step in viral replication. It is known that some compounds are reverse transcriptase inhibitors and are effective agents in the treatment of AIDS and similar diseases, e.g., azidothymidine or AZT.

Nucleotide sequencing of HIV shows the presence of a pol gene in one open reading frame [Ratner, L. et al., Nature, 313, 277(1985)]. Amino acid sequence homology provides evidence that the pol sequence encodes reverse transcriptase, an endonuclease and an HIV protease [Toh, H. et al., EMBO J. 4, 1267 (1985); Power, M.D. et al., Science, 231, 1567 (1986); Pearl, L.H. et al., Nature 329, 351 (1987)].

The compounds of this invention are inhibitors of HIV reverse transcriptase. Furthermore, the compounds of the present invention do not require bioactivation to be effective.

## BRIEF DESCRIPTION OF THE INVENTION

Novel compounds of formula A:

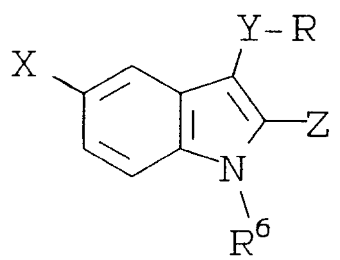

A

as herein defined, are disclosed. These compounds are useful in the inhibition of HIV reverse transcriptase, the prevention of infection by HIV, the treatment of infection by HIV and in the treatment of AIDS and/or ARC, either as compounds, pharmaceutically acceptable salts (when appropriate), pharmaceutical composition ingredients, whether or not in combination with other antivirals, anti-infectives, immunomodulators, antibiotics or vaccines. Methods of treating AIDS, methods of preventing infection by HIV, and methods of treating infection by HIV are also disclosed.

## DETAILED DESCRIPTION OF THE INVENTION AND PREFERRED EMBODIMENTS

This invention is concerned with the compounds of formula A described below, combinations thereof, or pharmaceutically acceptable salts or esters thereof, in the inhibition of HIV reverse transcriptase, the prevention or treatment of infection by HIV and in the treatment of the resulting acquired immune deficiency syndrome (AIDS). The compounds of this invention are those with structural formula A:

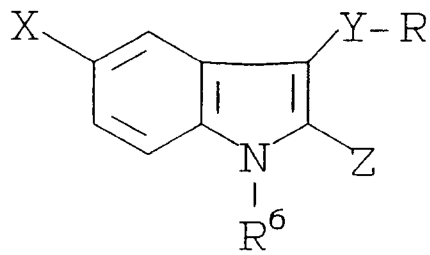

A

wherein

X is -H, -Cl, -F, -Br, $-NO_2$, -CN, or $-OR^2$;

Y is $-S(O)_n-$, $-CR^2R^2-$ or -O-, wherein n is zero, 1 or 2;

R is

1) hydrogen,
2) $-C_{1-5}$alkyl, unsubstituted or substituted with one or more of:
    a) $-C_{1-5}$alkyl,
    b) $-C_{1-5}$alkoxy,
    c) -OH, or
    d) aryl, unsubstituted or substituted with one or more of:
        i) $-C_{1-5}$alkyl,
        ii) $-C_{1-5}$alkoxy,
        iii) -OH, or
        iv) halogen,
3) aryl, unsubstituted or substituted with one or more of:
    a) $-C_{1-5}$alkyl, unsubstituted or substituted with one or more of:
        i) -OH or
        ii) $-C_{1-5}$alkoxy,
    b) $-C_{1-5}$alkoxy,
    c) -OH, or
    d) halogen, or
4) heterocycle, unsubstituted or substituted with one or more of:
    a) $-C_{1-5}$alkyl, unsubstituted or substituted with one or more of:
        i) -OH or
        ii) $-C_{1-5}$alkoxy,
    b) $-C_{1-5}$alkoxy, or
    c) -OH;

Z is

1)

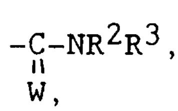

wherein W is O, S, -N-CN or $-N-OR^1$,

2)

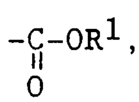

3)

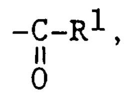

4)

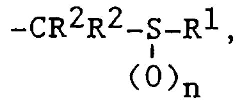

wherein n is defined above,
5) $-CR^2R^2NHR^4$,
6)

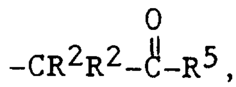

7) $-C_{1-3}$alkyl, unsubstituted or substituted with one or more of:
   a) aryl, unsubstituted or substituted with one or more of:
      i) $-C_{1-5}$alkyl,
      ii) $-C_{1-5}$alkoxy,
      iii) -OH or
      iv) halogen, or
   b) heterocycle, unsubstituted or substituted with one or more of:
      i) $-C_{1-5}$alkyl,
      ii) $-C_{1-5}$alkoxy, or
      iii) -OH, or
8) -CN;

$R^1$ is

1) hydrogen,
2) $-C_{1-5}$alkyl, unsubstituted or substituted with one or more of:
   a) $-C_{1-5}$alkyl,
   b) $-C_{1-5}$alkoxy,
   c) -OH,
   d) aryl, unsubstituted or substituted with one or more of:
      i) $-C_{1-5}$alkyl,
      ii) $-C_{1-5}$alkoxy, or
      iii) -OH, or
   e) heterocycle, unsubstituted or substituted with one or more of:
      i) $-C_{1-5}$alkyl,
      ii) $-C_{1-5}$alkoxy, or
      iii) -OH, or

3) aryl, unsubstituted or substituted with one or more of:
   a) $-C_{1-5}$alkyl, unsubstituted or substituted with one or more of:
      i) -OH or
      ii) $-C_{1-5}$alkoxy,
   b) $-C_{1-5}$alkoxy,
   c) -OH,
   d) halogen,
   e) -CN,
   f) $-NO_2$, or

g) $-NR^2R^2$; or
4) heterocycle, unsubstituted or substituted with one or more of:
a) $-C_{1-5}$alkyl, unsubstituted or substituted with one or more of:
i) -OH or
ii) $-C_{1-5}$alkoxy,
b) $-C_{1-5}$alkoxy, or
c) -OH;

$R^2$ is hydrogen or $C_{1-3}$alkyl;

$R^3$ is
1) $-C_{1-5}$alkyl, unsubstituted or substituted with one or more of:
a) $-C_{1-5}$alkyl,
b) $-C_{1-5}$alkoxy, unsubstituted or substituted with -OH,
c) -OH,
d)

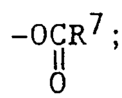

e) $-COOR^2$
f) aryl, unsubstituted or substituted with one or more of:
i) $-C_{1-5}$alkyl, unsubstituted or substituted with one or more of -OH,
ii) $-C_{1-5}$alkoxy,
iii) -OH, or
iv) halogen,
g) heterocycle, unsubstituted or substituted with one or more of:
i) $-C_{1-5}$alkyl,
ii) $-C_{1-5}$alkoxy,
iii) -OH,
iv) $C_{1-3}$alkyl-$NR^2R^2$
h) $-NR^2R^2$,
i) $-C_{3-6}$cycloalkyl,
2) aryl, unsubstituted or substituted with one or more of:
a) $-C_{1-5}$alkyl,
b) $-C_{1-5}$alkoxy,
c) -OH,
d) halogen,
3) heterocycle, unsubstituted or substituted with one or more of:
i) $-C_{1-5}$alkyl,
ii) $-C_{1-5}$alkoxy,
iii) -OH,
4) $-C_{1-5}$alkoxy,
5) -OH,
6) $-C_{3-6}$cycloalkyl, or
7) hydrogen;

$R^4$ is
1) $R^1$ or
2)

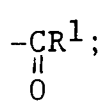

$R^5$ is
1) $R^1$,
2) $-C_{1-5}$alkoxy,

3) $-NHR^1$, or
4) heterocycle, unsubstituted or substituted with one or more of:
i) $-C_{1-5}$alkyl,
ii) $-C_{1-5}$alkoxy,
iii) -OH;

$R^6$ is
1) hydrogen,
2)

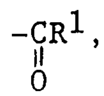

or
3)

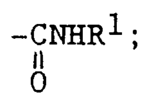

and

$R^7$ is
1) aryl, unsubstituted or substituted with one or more of -Cl, -Br, -OH, $-OCH_3$, or -CN, or
2) $-C_{1-5}$alkyl, unsubstituted or substituted with one or more of -OH or $-NR^2R^2$, with the proviso that when X is -H, Y is -S, R is unsubstituted phenyl and $R^6$ is -H,
Z is not

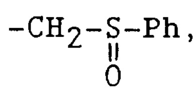 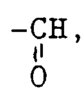

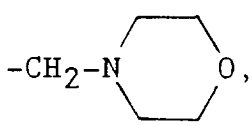 or 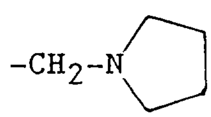 ;

or a pharmaceutically acceptable salt or ester thereof.

A preferred embodiment of this invention encompasses compounds of Formula A further limited to:

X is -H, -Cl or -F;
Y is $-S(O)_n-$ or $-CH_2-$;
R is -Ph, or -tolyl;
$R^6$ is -H; and
Z is

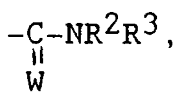 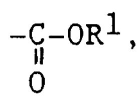 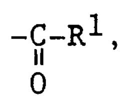

or

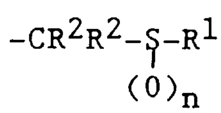

$-C_{1-3}$alkyl substituted with heterocycle.

A second, more preferred embodiment is further limited to compounds wherein

X is -H or -Cl;

Y is $-S(O)_n-$;

R is -Ph, or -tolyl;

$R^6$ is -H; and

Z is

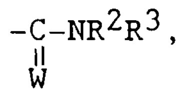

wherein $R^2$ is -H and W is -O or -S, or

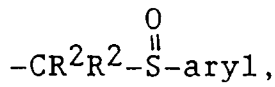

wherein the aryl group is unsubstituted or substituted with one or more of $C_{1-5}$ alkyl.

A third, most preferred embodiment is further limited to compounds wherein

X is -Cl;

Y is $-S(O)_n-$;

R is -Ph;

$R^6$ is -H;

Z is

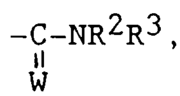

wherein $R^2$ is -H, and W is -O or -S; and

$R^3$ is

1) $-C_{1-5}$ alkyl, unsubstituted or substituted with one or more of
   a) $-C_{1-5}$ alkoxy, unsubstituted or substituted with -OH,
   b) -OH,
   c)

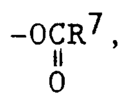

   d) aryl, unsubstituted or substituted with one or more of:
      i) $-C_{1-5}$ alkyl, unsubstituted or substituted with one or more of -OH,
      ii) $-C_{1-5}$ alkoxy,
      iii) -OH,
   e) heterocycle, unsubstituted or substituted with one or more of:
      i) $-C_{1-5}$ alkyl,
      ii) $-C_{1-5}$ alkoxy, or
      iii) -OH,
2) heterocycle, unsubstituted or substituted with one or more of:
   i) $-C_{1-5}$ alkyl,
   ii) $-C_{1-5}$ alkoxy, or
   iii) -OH, or
3) hydrogen.

The most preferred compounds of this invention are compounds 1 through 18, shown below.

Compound 1:

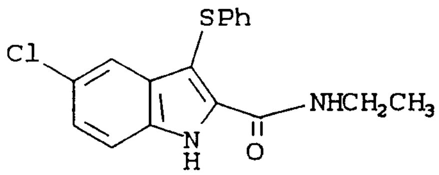

N-ethyl-5-chloro-3-phenylthioindole-2-carboxamide

Compound 2:

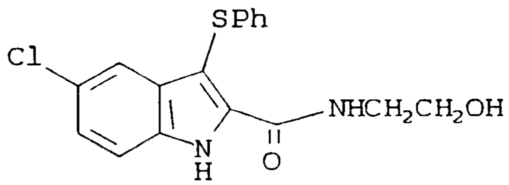

N-2-hydroxyethyl-5-chloro-3-phenylthioindole-2-carboxamide

Compound 3:

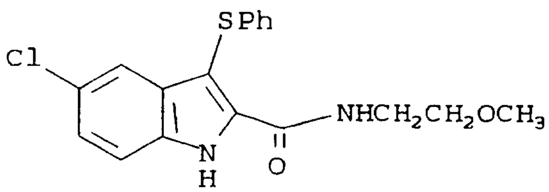

N-2-methoxyethyl-5-chloro-3-phenylthioindole-2-carboxamide

Compound 4:

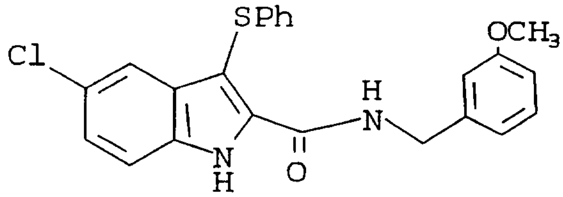

N-3-methoxybenzyl-5-chloro-3-phenylthioindole-2-carboxamide

Compound 5:

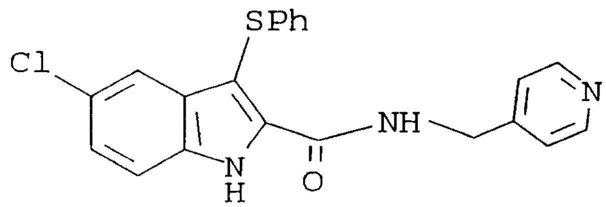

N-4-pyridymethyl-5-chloro-3-phenylthioindole-2-carboxamide

Compound 6:

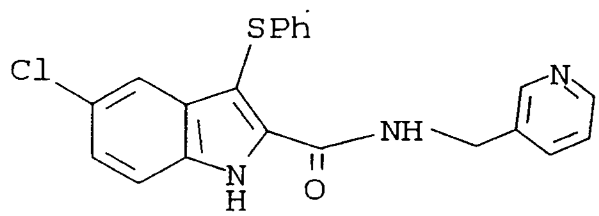

N-3-pyridylmethyl-5-chloro-3-phenylthioindole-2-carboxamide

Compound 7:

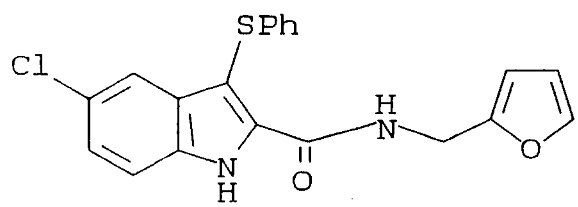

N-2-furanylmethyl-5-chloro-3-phenylthioindole-2-carboxamide

Compound 8:

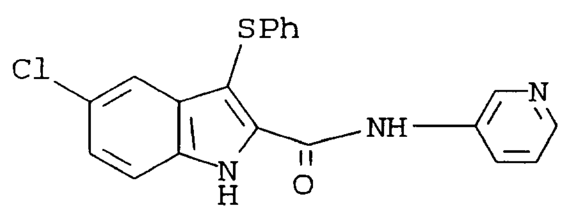

N-3-pyridyl-5-chloro-3-phenylthioindole-2-carboxamide

Compound 9:

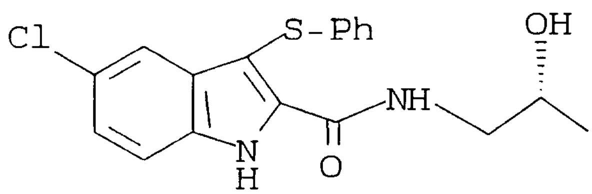

N-[1-(2(R)-hydroxypropyl)]-5-chloro-3-phenylthioindole-2-carboxamide

Compound 10:

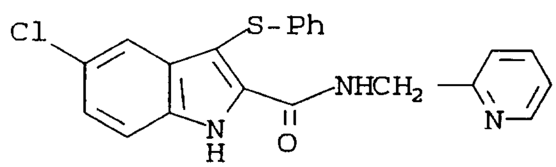

N-(2-pyridyl)methyl-5-chloro-3-phenylthioindole-2-carboxamide

Compound 11:

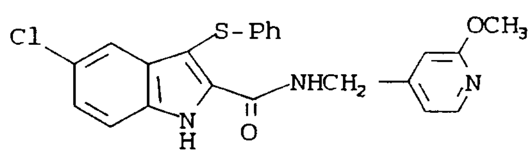

N-(3-methoxy-4-pyridyl)methyl-5-chloro-3-phenylthioindole-2-carboxamide

Compound 12:

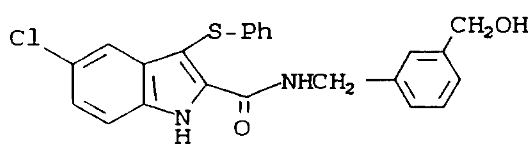

N-(3-hydroxymethyl)benzyl-5-chloro-3-phenylthioindole-2-carboxamide

Compound 13:

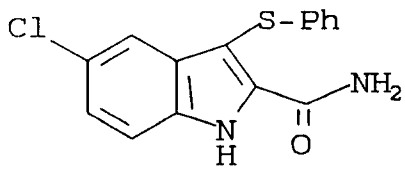

5-chloro-3-phenylthioindole-2-carboxamide

Compound 14:

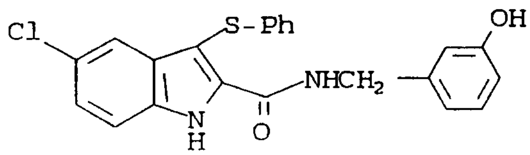

N-(3-hydroxybenzyl)-5-chloro-3-phenylthioindole-2-carboxamide

Compound 15:

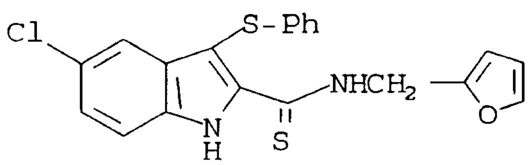

N-2-furanylmethyl-5-chloro-3-phenylthioindole-2-thiocarboxamide

Compound 16:

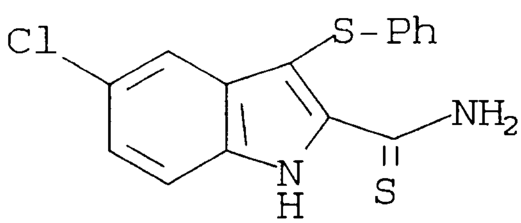

5-chloro-3-phenylthioindole-2-thiocarboxamide

Compound 17:

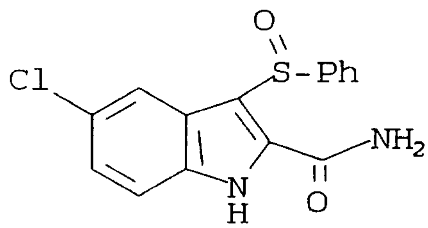

5-chloro-3-phenylsulfinylindole-2-carboxamide

Compound 18:

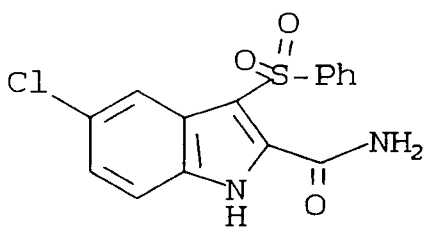

5-chloro-3-phenylsulfonylindole-2-carboxamide.

The compounds of the present invention may have asymmetric centers and occur as racemates, racemic mixtures, individual diastereomers, or enantiomers, with all isomeric forms being included in the present invention.

When any variable (e.g., aryl, heterocycle, $R^1$, $R^2$, $R^3$, etc.) occurs more than one time in any constituent or in formula A of this invention, its definition on each occurrence is independent of its definition at every other occurrence. Also, combinations of substituents and/or variables are permissible only if such combinations result in stable compounds.

As used herein except where noted, "alkyl" is intended to include both branched- and straight-chain saturated aliphatic hydrocarbon groups having the specified number of carbon atoms; "alkoxy" represents an alkyl group of indicated number of carbon atoms attached through an oxygen bridge. "Halogen" or "halo" as used herein, means fluoro, chloro, bromo and iodo.

As used herein, with exceptions as noted, "aryl" is intended to mean any stable monocyclic, bicyclic or tricyclic carbon ring of up to 7 members in each ring, wherein at least one ring is aromatic. Examples of such aryl elements include phenyl, naphthyl, tetrahydronaphthyl, biphenyl.

The term heterocycle or heterocyclic, as used herein except where noted, represents a stable 5- to 7-membered monocyclic or stable 8- to 11-membered bicyclic heterocyclic ring which is either saturated or unsaturated, and which consists of carbon atoms and from one to three heteroatoms selected from the group consisting of N, O and S, and wherein the nitrogen and sulfur heteroatoms may optionally be oxidized, and the nitrogen heteroatom may optionally be quaternized, and including any bicyclic group in which any of the above-defined heterocyclic rings is fused to a benzene ring. The heterocyclic ring may be attached at any heteroatom or carbon atom which results in the creation of a stable structure. Examples of such heterocyclic elements include piperidinyl, piperazinyl, 2-oxopiperazinyl, 2-oxopiperidinyl, 2-oxopyrrolidinyl, 2-oxoazepinyl, azepinyl, pyrrolyl, 4-piperidonyl, pyrrolidinyl, pyrazolyl, pyrazolidinyl, imidazolyl, imidazolinyl, imidazolidinyl, pyridyl, pyrazinyl, pyrimidinyl, pyridazinyl, oxazolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, thiadiazoyl, benzopyranyl, benzothiazolyl, benzoxazolyl, furyl, tetrahydrofuryl, benzofuranyl, tetrahydropyranyl, thienyl, benzothienyl, thiamorpholinyl, thiamorpholinyl sulfoxide, thiamorpholinyl sulfone, and oxadiazolyl.

Further abbreviations that may appear in this application are as follows:

| | |
|---|---|
| Me | methyl |
| Et | ethyl |
| Ph | phenyl |
| BuLi | butyllithium |
| n-$Bu_3P$ | tri-n-butyl phosphine |
| LAH | lithium aluminum hydride |
| DMF | dimethylformamide |
| THF | tetrahydrofuran |
| $Et_3N$ | tri-ethylamine |
| MMPP | monoperoxyphthalic acid, magnesium salt |
| BOP-reagent | benzotriazol-1-yloxytris(dimethylamino)phosphonium hexafluorophosphate |
| mp or m.p. | melting point |

The pharmaceutically-acceptable salts of the novel compounds of this invention that are capable of salt formation (in the form of water- or oil- soluble or dispersible products) include the conventional non-toxic salts or the quaternary ammonium salts of these compounds, which are formed, e.g., from inorganic or organic acids or bases. Examples of such acid addition salts include acetate, adipate, alginate, aspartate, benzoate, benzenesulfonate, bissulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Also, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others. Esters are also encompassed by the present invention, and include those which would readily occur to the skilled artisan, for example, $C_{1-4}$ alkyl esters.

Schemes I-VII for preparing the novel compounds of this invention are presented below. Tables I - VII which follow the schemes illustrate the compounds that can be synthesized by Schemes I-VII, but Schemes I - VII are not limited by the compounds in the tables nor by any particular substituents employed in the schemes for illustrative purposes. The examples specifically illustrate the application of the following schemes to specific compounds.

Scheme I, below, is a general route for synthesizing, e.g., the compounds shown in Table I, infra. The substituent groups (e.g., X, R, $R^1$, etc.) employed in Scheme I correspond to the substituent groups as defined in Table I, but Scheme I is not limited by the defined substituents or compounds of Table I.

## SCHEME I

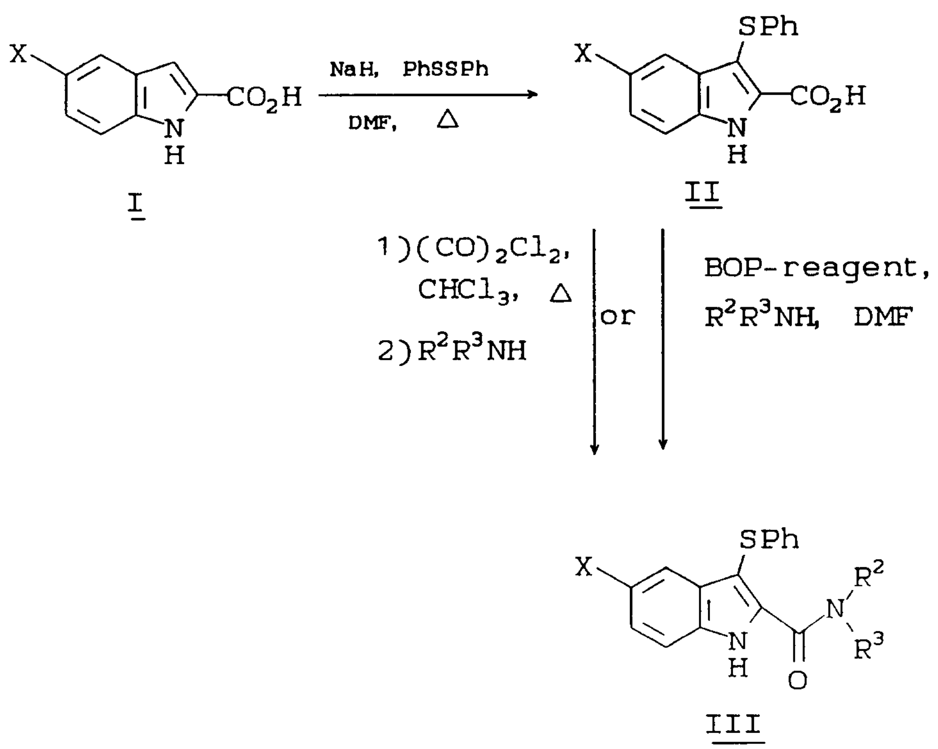


As shown in Scheme I, commercially available indole-2-carboxylic acid (or 5-chloro, 5-fluoro or 5-methoxyindole-2-carboxylic acid) I is treated with an excess of sodium hydride in dimethylformamide in the presence of an aryl disulfide such as phenyldisulfide at 0°C to 60°C, according to the general procedure described by Atkinson, et al. in Synthesis, p. 480-481 (1988). The resulting product II is reacted with oxalyl chloride in refluxing chloroform for about 30 minutes to 1 hour to produce the corresponding acid chloride which is then reacted with a primary or secondary amine in chloroform at 0°C to 20°C to give the amide III. Alternatively, amide III can be produced directly from II by treatment with BOP reagent (benzotriazol-1-yloxytris-(dimethylamino)phosphonium hexafluorophosphate) in the presence of the desired primary or secondary amine and triethylamine, in a solvent such as dimethylformamide. Saponification of ethyl 5-chloro-3-benzylindole-2-carboxylate (prepared as described below) by methods familiar to those skilled in the art, yields 5-chloro-3-benzylindole-2-carboxylic acid, which can be converted to the desired amides in the manner described for the synthesis of amides III.

The compounds shown in Table II infra, are generally synthesized as in Scheme I, except $R^1OH$ is used in place of $R^2R^3NH$, as depicted in Scheme II below. The substituent groups employed in Scheme II correspond to the substituent groups as defined in Table II, but Scheme II is not limited by the defined substituents or compounds of Table II.

SCHEME II

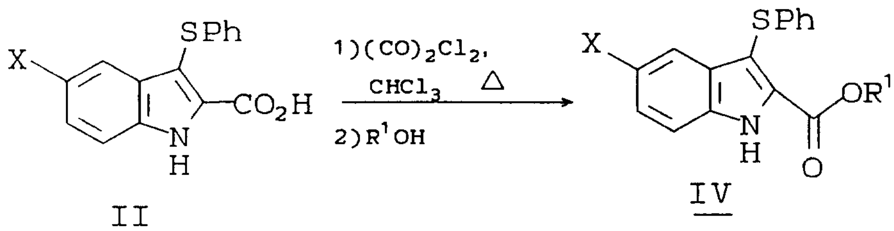


II

IV

As shown in Scheme II, 3-phenylthioindole-2-carboxylic acid II can be converted to the corresponding acid chloride with oxalyl chloride in refluxing chloroform, and reacted with an alcohol to give the ester IV. The compound ethyl 5-chloro-3-benzylindole-2-carboxylatewas prepared according to the procedure described by Inaba, et al., in Chem. Pharm. Bull., 24, p. 1076-1082 (1976).

Scheme III, below, is a general route for synthesizing, e.g., the compounds shown in Table III, infra. The substituent groups employed in Scheme III correspond to the substituent groups as defined in Table III, but Scheme III is not limited by the defined substituents or compounds of Table III.

SCHEME III

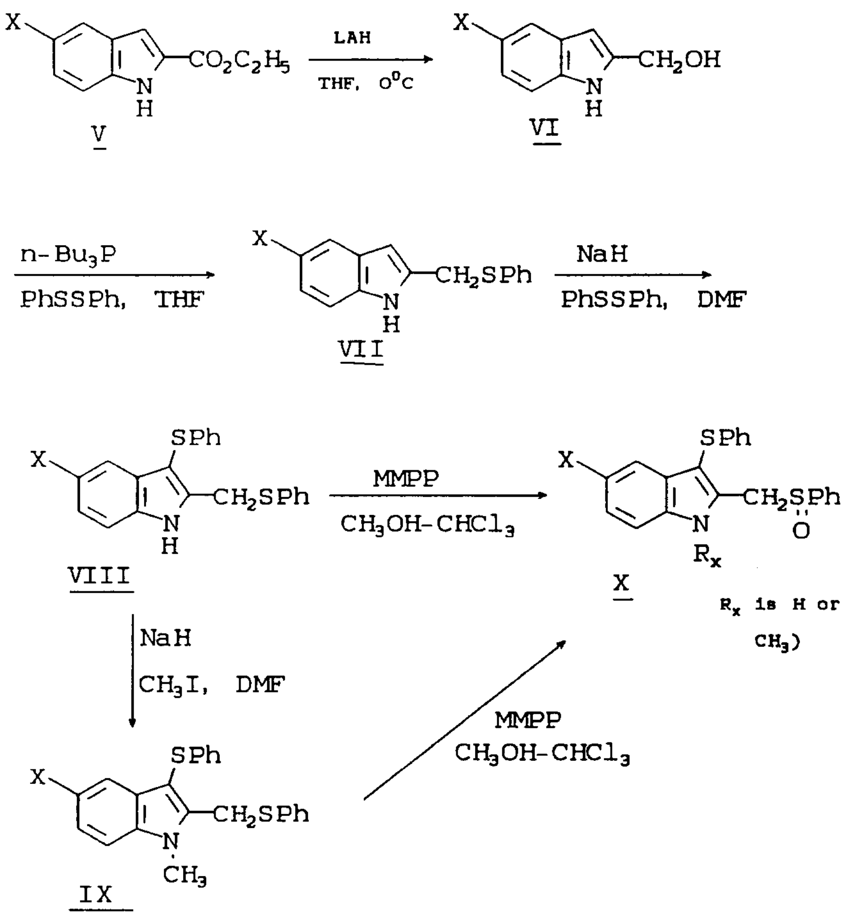


As shown in Scheme III, commercially available ethyl indole-2-carboxylate (compound V wherein X is -H) or ethyl 5-chloroindole-2-carboxylate (compound V wherein X is -Cl), was reduced to the primary alcohol VI with an excess of lithium aluminum hydride in tetrahydrofuran at 0 °C. Compound VI was converted to the sulfide VII by treatment with an excess of tri-n-butylphosphine and an aryldisulfide such as phenyldisulfide in tetrahydrofuran at 0 °C to 20 °C for 6-24 hours. Reaction of sulfide VII with sodium hydride, an aryldisulfide such as phenyldisulfide, in dimethylformamide at 0 °C to 20 °C for 1 to 18 hours produces bis-sulfide VIII. Aryl disulfides which were not commercially available were obtained by oxidation of the commercially available aryl mercaptan with dimethyl sulfoxide and iodine, according to the procedure described by Orville G. Lowe in J. Org. Chem., 40, p. 2096-2098 (1975). Compound VIII can be N-alkylated, if desired, by methods familiar to those trained in the art, e.g., by treatment with sodium hydride in dimethylformamide at 0 °C in the presence of an alkylating agent such as iodomethane, to give compound IX. Thereafter, compound VIII (or IX) is treated with one equivalent of peracid such as monoperoxyphthalic acid, magnesium salt (MMPP), or meta-chloroperoxy-benzoic acid in methanol or chloroform-methanol at 0 °C for 30 minutes to 3 hours, to give predominately sulfoxide X.

Schemes IV-A and IV-B , below, show a general route for synthesizing, e.g., the compounds shown in Tables IV-A and IV-B, infra. The substituent groups employed in Schemes IV-A and IV-B, respectively, correspond to the substituent groups as defined in Tables IV-A and IV-B, respectively, but Schemes IV-A and IV-B are not limited by the defined substituents or compounds of Tables IV-A and IV-B.

SCHEME IV-A

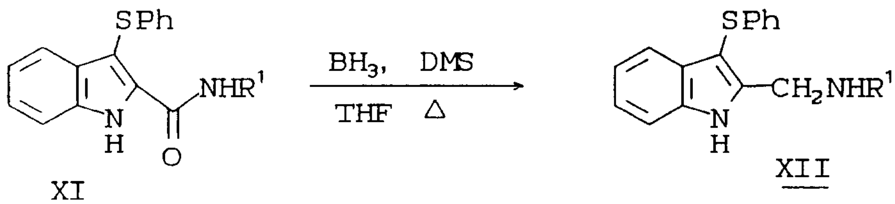


XI XII

SCHEME IV-B

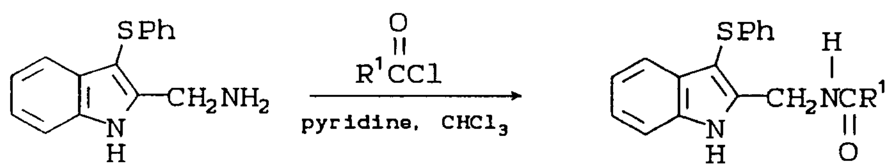


XII-A XIII

As shown in Scheme IV-A, 3-phenylthioindole-2-carboxamides XI can be reduced to primary or secondary amines XII by reaction with an excess of borane-dimethylsulfide complex in refluxing tetrahydrofuran for 6-24 hours. As shown in Scheme IV-B, the primary amine XII-A can be acylated with an acid chloride, such as benzoyl chloride, in chloroform in the presence of pyridine, to give the amide XIII.

Scheme V, below, is a general route for synthesizing, e.g., the compounds shown in Table V-A and Table V-B, infra. The substituent groups employed in Scheme V correspond to the substituent groups as defined in Tables V-A and V-B, but Scheme V is not limited by the defined substituents or compounds of Tables V-A and V-B.

SCHEME V

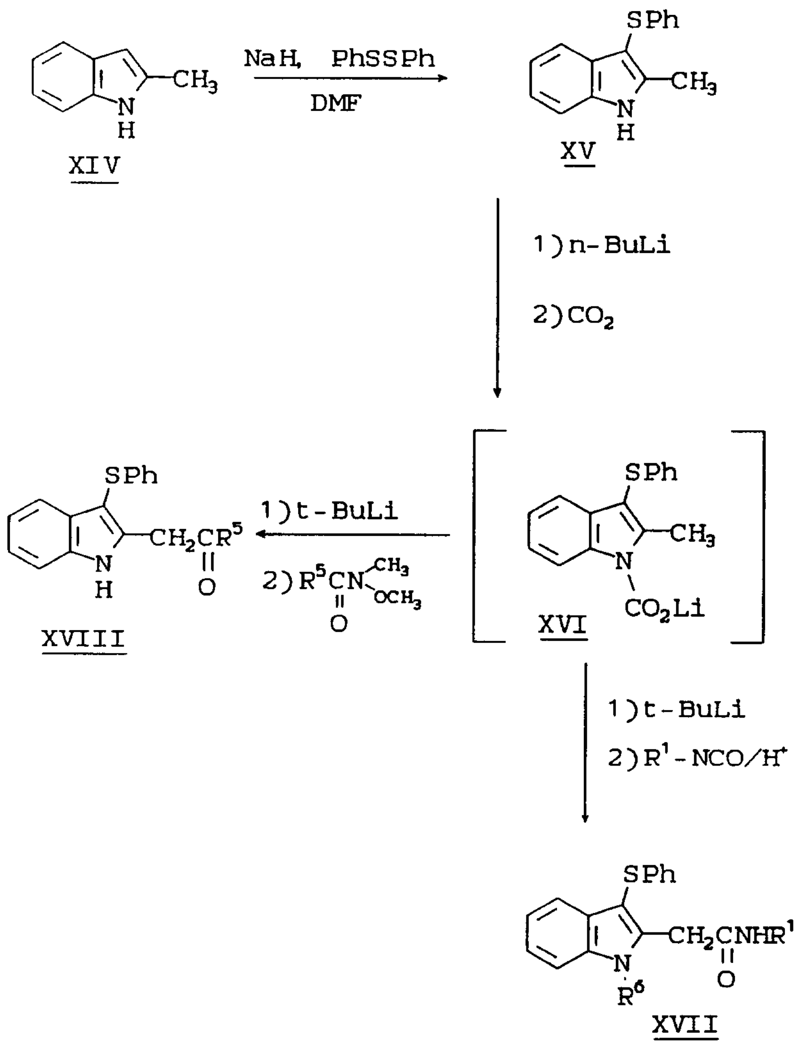


As shown in Scheme V, commercially available 2-methylindole XIV can be treated with sodium hydride in dimethylformamide in the presence of an aryldisulfide such as phenyldisulfide to give compound XV. Compound XV can be converted to the monoanion with n-butyllithium in tetrahydrofuran at -78°C, and then reacted with carbon dioxide to give carboxylate XVI. The dianion formed by the reaction of XVI with t-butyllithium could be reacted with an isocyanate, such as phenylisocyanate, to give a mixture of monoacylated product and diacylated product XVII (see Table V-B). Alternatively, the dianion formed by the reaction of XVI with t-butyllithium could be reacted with an N-methoxy-N-methyl amide such as N-methoxy-N-methyl-furan-2-carboxamide (prepared in a manner familiar to those skilled in the art, e.g., by the methods described in Scheme 1) to produce ketones XVIII. The methodology described above is essentially that used by A. J. Katritsky and K. Akutagawa to prepare 2-indoleacetic acids, and is published in J. Am. Chem. Soc., 108, 6808 (1986).

Scheme VI, below, is a general route for synthesizing, e.g., the compounds shown in Table VI, infra. The substituent groups employed in Scheme VI correspond to the substituent groups as defined in Table VI, but Scheme VI is not limited by the defined substituents or compounds of Table VI.

SCHEME VI

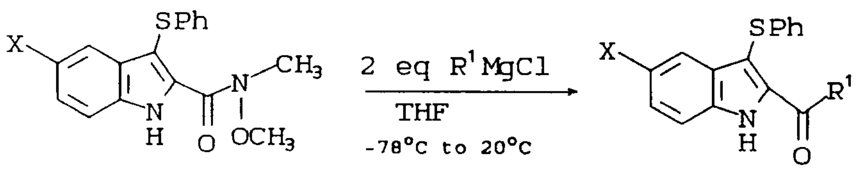


XIX XX

As shown in Scheme VI, N-methoxy-N-methyl-3-phenylthioindole-2-carboxamide XIX (or N-methoxy-N-methyl-5-chloro-3-phenylthioindole-2-carboxamide) (prepared as in Scheme 1) can be reacted with Grignard reagents (wherein $R^1$ is not hydrogen) such as phenylmagnesium chloride, in tetrahyrodrofuran at -78 ° C to 20 ° C for 18-48 hours, or XIX can be reacted with other organometallic reagents well known in the art to one of ordinary skill, to produce ketones XX.

The compounds shown in Table VII infra, can generally be synthesized by those of ordinary skill in the art according to methods described in Schemes I through VI, with the exception of 2-(2-benzoxazol-2-ylethyl)-3-phenylthioindole (compound XXIV), the synthesis of which is described below in Scheme VII.

SCHEME VII

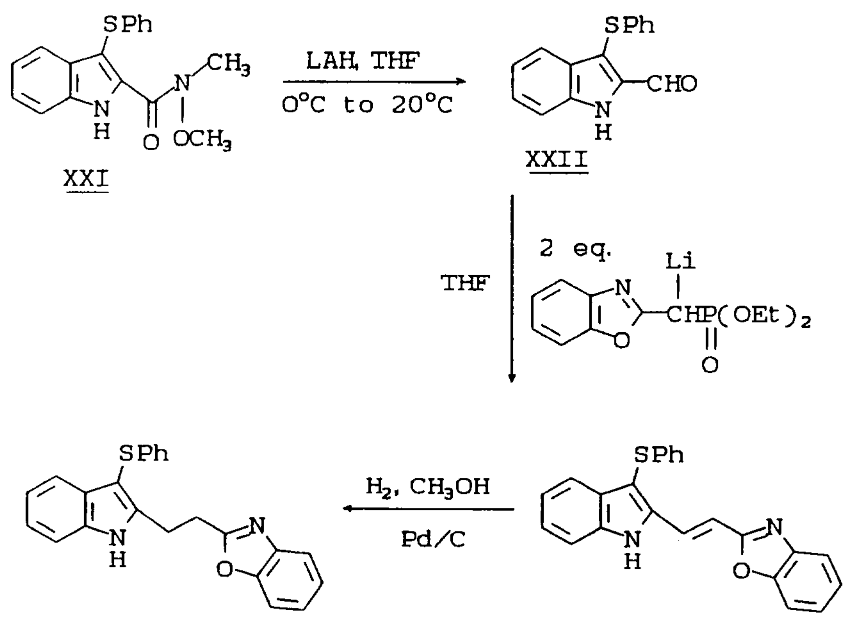


XXI XXII

XXIV XXIII

As shown in Scheme VII, N-methoxy-N-methyl-3-phenylthioindole-2-carboxamide XXI can be reduced to aldehyde XXII with lithium aluminum hydride in tetrahydrofuran at 0 ° C to 20 ° C for 2-4 hours. Aldehyde XXII could be reacted with the lithium salt of [(benzoxal-2-yl)methyl]diethyl-phosphonate to produce olefin

XXIII, which is then hydrogenated in the presence of 10% palladium on charcoal in methanol under one atmosphere of hydrogen to give compound XXIV.

The compound 5-chloro-2-cyano-3-phenylthioindole in Table VII can be prepared by dehydro-sulfurization of 5-chloro-3-phenylthioindole-2-thiocarboxamide with, e.g., $Hg(OAc)_2$.

Using methods well-known to those skilled in the art, compounds of formula A where Y is -SO- or $-SO_2-$ can be synthesized by treatment of compounds where Y is -S- with a suitable oxidizing agent such as, for example, meta-chloroperoxybenzoic acid (MCPBA), sodium periodate or hydrogen peroxide in an appropriate solvent such as MeOH, $CHCl_3$ or acetic acid, or potassium persulfate in a solvent such as $MeOH/H_2O$.

TABLE I

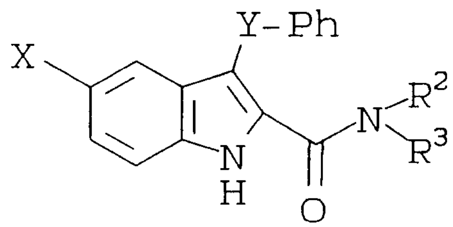

| X | Y | $R^2$ | $R^3$ | m p. |
|---|---|---|---|---|
| H | S | $CH_3$ | $CH_3$ | 194-195°C |
| H | S | H | $CH_2Ph$ | 179-181°C |
| H | S | H | Ph | 194-196°C |
| H | S | H | $n-C_4H_9$ | 161-163°C |
| H | S | H | $CH_2CH_2Ph$ | 147-149°C |
| Cl | S | $CH_3$ | $OCH_3$ | 57-59°C |
| Cl | S | H | 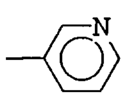 | 255-256°C |
| H | S | H | $CH_3$ | 197-199°C |

TABLE I, Cont'd

| X | Y | $R^2$ | $R^3$ | m p. |
|---|---|---|---|---|
| Cl | S | H | $nC_3H_7$ | 210-212°C |
| Cl | S | H | 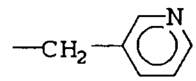 | 240-241°C |
| Cl | S | H | 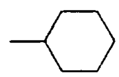 | 255-256°C |
| F | S | H | 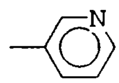 | 239-241°C |
| Cl | S | H | 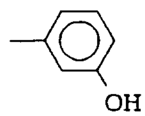 | 232-233°C |
| Cl | $CH_2$ | H | 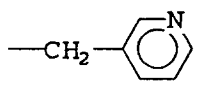 | 243-244°C |
| Cl | S | H | 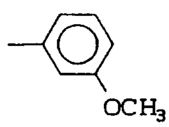 | 221°C |
| Cl | S | H | 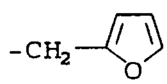 | 214°C |

TABLE I, Cont'd

| X | Y | $R^2$ | $R^3$ | m p. |
|---|---|---|---|---|
| Cl | S | H | $-CH_2CH_2CH_2OH$ | 215°C |
| Cl | S | H | 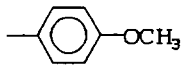 | 229°C |
| Cl | S | H | $CH_3$ | 220-221°C |
| Cl | S | H | $CH_2CH_2CH_2OCH_3$ | 170-171°C |
| Cl | S | H | 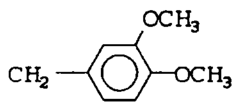 | 184°C |
| Cl | S | H | 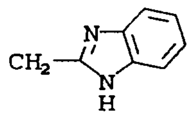 | 259-260°C (HCl salt) |
| Cl | S | H | 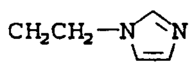 | 256°C (HCl salt) |
| Cl | S | H | $CH(CH_3)_2$ | 193-194°C |
| Cl | S | $CH_3$ | Ph | 191-192°C |

TABLE I, Cont'd

| X | Y | $R^2$ | $R^3$ | m p. |
|---|---|---|---|---|
| Cl | S | H | $CH_2CH_2CH_2N(CH_3)_2$ | 229-230°C (HCl salt) |
| Cl | S | H | $C_2H_5$ | 210-211°C |
| Cl | S | H | Ph | 245-246°C |
| Cl | S | H | $CH_2$ 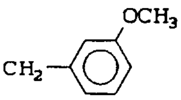 | 172°C |
| Cl | S | H | $CH_2CH_2CH_2$- 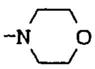 | 162-164°C |
| Cl | S | H | $CH_2$ 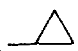 | 219-219.5°C |
| Cl | S | H | $CH_2Ph$ | 222°C |
| Cl | S | H | $CH_2$ 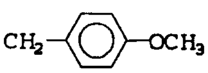 | 198°C |
| Cl | S | H | $CH_2CH_2OCH_2CH_2OH$ | 161.5-162.5°C |

TABLE I, Cont'd

| X | Y | $R^2$ | $R^3$ | m p. |
|---|---|---|---|---|
| Cl | S | H | 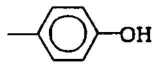 | >300°C |
| Cl | S | H | $CH_2CH_2OCH_3$ | 216-217. 5°C |
| Cl | S | H | $CH_2CH_2OC_2H_5$ | 165. 5-167°C |
| Cl | S | H | 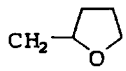 | 182-183°C |
| Cl | S | H | 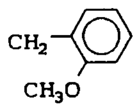 | 201°C |
| Cl | S | H | 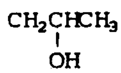 | 205°C |
| Cl | S | H | 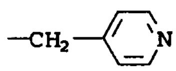 | 228-229°C |
| Cl | S | H | $CH_2CH_2OH$ | 222-223. 5°C |
| Cl | S | H | 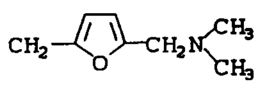 | 151-152°C |

TABLE I, Cont'd

| X | Y | $R^2$ | $R^3$ | mp |
|---|---|---|---|---|
| Cl | S | H | $OCH_3$ | 179-180°C |
| Cl | S | H | $-CH-CH_2CH_3$<br>$CH_2OH$ | 153-154°C |
| Cl | S | H | $CH_2\overset{O}{\overset{\|}{C}}OC_2H_5$ | 215-215. 2°C |
| Cl | S | H | $-CHCH_2OCH_3$<br>$CH_3$ | 168-169°C |
| Cl | S | H | 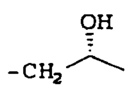 | 202-203°C |
| Cl | S | H | 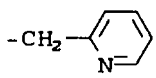 | 209-210°C |

TABLE I, Cont'd

| X | Y | $R^2$ | $R^3$ | mp |
|---|---|---|---|---|
| Cl | S | H | 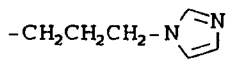 •HCl | 188-189°C |
| Cl | S | H | 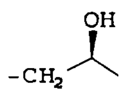 | 205-206°C |
| Cl | S | H | 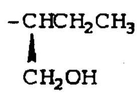 | 138-139°C |
| Cl | S | H | 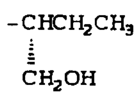 | 137.5-139°C |
| Cl | S | H | 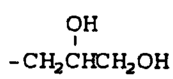 | 219-221°C |
| Cl | S | H | 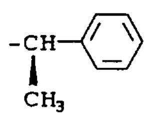 | 208-210°C |
| Cl | S | H | 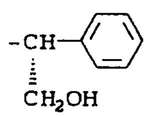 | 223-226°C |

TABLE I, Cont'd

| X | Y | $R^2$ | $R^3$ | mp |
|---|---|---|---|---|
| Cl | S | H | 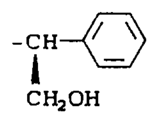 | 223-226°C |
| Cl | S | H | 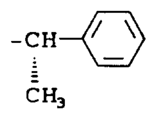 | 208-210°C |
| Cl | S | H | 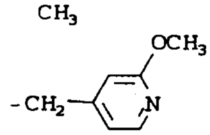 | 227-228°C |
| Cl | S | H | 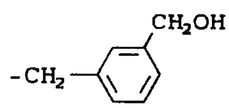 | 229-230°C |
| Cl | S | H | 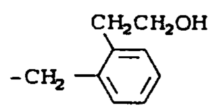 | 217-219°C |
| Cl | S | H | 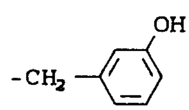 | 214-216°C |
| Cl | S | H | 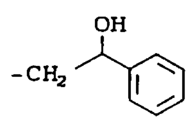 | 193-195.5°C |
| Cl | S | H | -H | 213-215°C |

TABLE I, Cont'd

| X | Y | $R^2$ | $R^3$ | mp |
|---|---|---|---|---|
| Cl | $SO_2$ | H | H | 255-257°C |
| Cl | $SO_2$ | H | 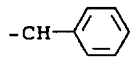 | |
| Cl | $SO_2$ | H | 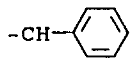 | |
| Cl | $SO_2$ | H | $-CH_2CH_2OH$ | |
| Cl | $SO_2$ | H | 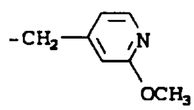 | |

TABLE II

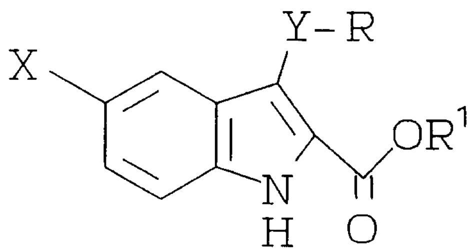

| X | Y | R | $R^1$ | mp |
|---|---|---|---|---|
| H | S | Ph | $CH_3$ | 179-180°C |
| H | S | 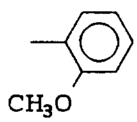 | $CH_3$ | 195-197°C |
| $OCH_3$ | S | Ph | $CH_3$ | 211-212°C |
| Cl | S | Ph | $CH_3$ | 193-196°C |
| Cl | S | Ph | $CH_2Ph$ | 154-155°C |
| Cl | S | Ph | $C_2H_5$ | 163-164°C |
| Cl | $CH_2$ | Ph | $C_2H_5$ | 196-197°C |
| F | S | Ph | $C_2H_5$ | 149°C |

## TABLE III

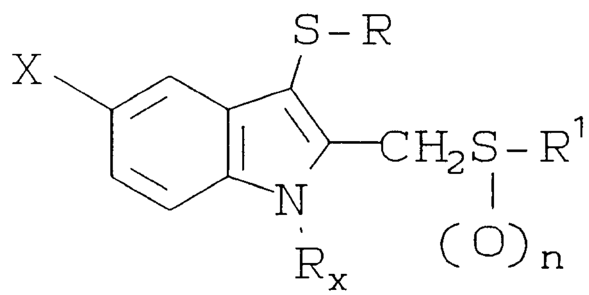

| X | R | $R^1$ | n | $R_x$ | mp |
|---|---|---|---|---|---|
| H | Ph | Ph | 1 | H | 71-74°C |
| H | Ph | Ph | 0 | H | --- |
| H | Ph | Ph | 2 | H | 168-169°C |
| H | 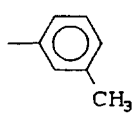 | Ph | 1 | H | 166-167°C |
| H | 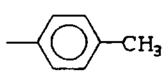 | Ph | 1 | H | 164.5-166.5°C |
| $OCH_3$ | Ph | Ph | 1 | H | 70-80°C |

TABLE III, Cont'd

| X | R | $R^1$ | n | $R_x$ | mp |
|---|---|---|---|---|---|
| H | 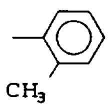 | Ph | 1 | H | 171.5-172.5°C |
| H | Ph | Ph | 0 | $CH_3$ | 98-99°C |
| H | Ph | Ph | 1 | $CH_3$ | 177-178°C |
| H | Ph | 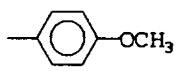 | 1 | H | --- |
| H | Ph | $CH_3$ | 1 | H | 164-168°C |
| H | Ph | 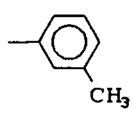 | 0 | H | --- |

TABLE III, Cont'd

| X | R | $R^1$ | n | $R_x$ | mp |
|---|---|---|---|---|---|
| H | Ph | 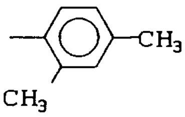 | 0 | H | --- |
| H | Ph | 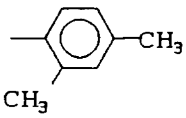 | 1 | H | --- |
| H | Ph | 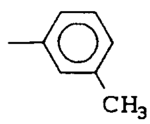 | 1 | H | --- |
| H | Ph | 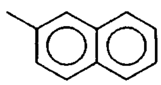 | 1 | H | --- |
| H | Ph | 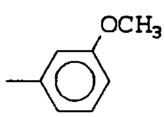 | 1 | H | --- |

TABLE III, Cont'd

| X | R | $R^1$ | n | $R_x$ | mp |
| --- | --- | --- | --- | --- | --- |
| H | Ph | 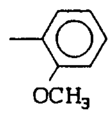 | 1 | H | 138-140°C |
| H | Ph | 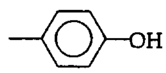 | 0 | H | --- |
| H | Ph | 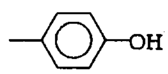 | 1 | H | 219-220°C |
| Cl | Ph | Ph | 1 | H | 158-162°C |
| H | Ph | 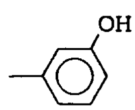 | 1 | H | --- |

TABLE IV-A

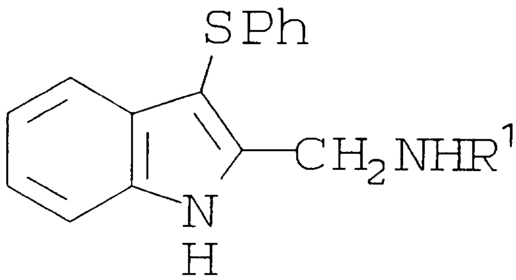

| $R^1$ | m p. |
| --- | --- |
| $-CH_2Ph$ | 199-201°C |
| -Ph | 167-170°C |
| $-n-C_4H_9$ | 177-179°C |
| -H | --- |

TABLE IV-B

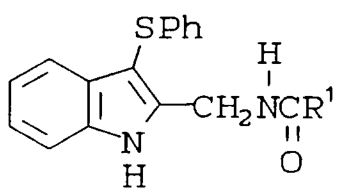

| $R^1$ | m p. |
| --- | --- |
| $-CH_3$ | --- |
| -Ph | 64-65°C |

TABLE V-A

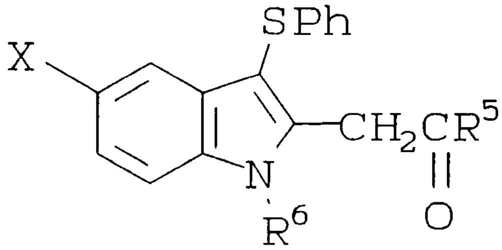

| X | $R^5$ | $R^6$ | mp |
|---|---|---|---|
| H | $-CH_2Ph$ | H | --- |
| H | -Ph | H | 154-155°C |
| H | $-CH_3$ | H | 101.5-103.5°C |
| H | 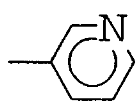 | H | 87-90°C |
| H | 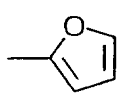 | H | 127-129°C |
| Cl | $-OC_2H_5$ | H | 99-103°C |

TABLE V-B

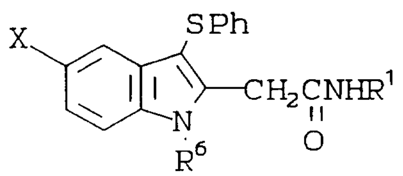

| X | $R^1$ | $R^6$ | mp |
|---|---|---|---|
| H | -Ph | H | 66-68°C |
| H | -Ph | 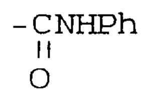 | 123-125°C |

TABLE VI

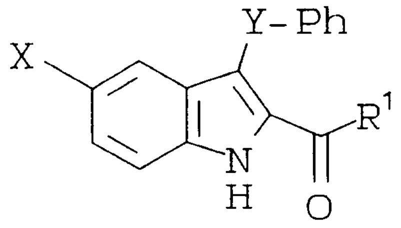

| X | Y | $R^1$ | mp |
|---|---|---|---|
| Cl | S | -Ph | 154-155°C |
| Cl | S | $-CH_2Ph$ | 219-220°C |
| H | S | -Ph | 121.5-123°C |
| H | S | 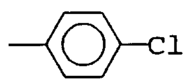 | 142-149°C |
| Cl | S | $-C_2H_5$ | 196-197°C |
| Cl | S | 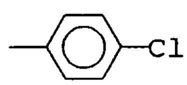 | 214-215°C |
| Cl | S | $-CH_3$ | 178°C |
| Cl | $CH_2$ | $-C_2H_5$ | 203-205°C |
| Cl | $CH_2$ | -Ph | 161-163°C |

TABLE VII

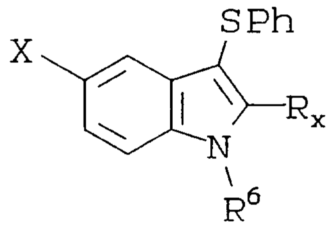

| X | $R_x$ | $R^6$ | mp |
|---|---|---|---|
| H | $-CH_3$ | H | 128-130°C |
| H | $-CH_3$ | -CNHPh<br>‖<br>O | 157-159°C |
| H | $-CH_3$ | -CPh<br>‖<br>O | 124-125°C |
| H | $-CH_2CH_2Ph$ | H | 117-120.5°C |
| H | $-CH_2CH_2-$ 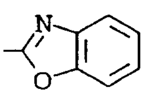 | H | 192-193°C |
| Cl | -CN | H | 172-174°C |
| Cl | $-C-NHCH_2-$ 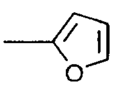<br>‖<br>S | H | 143-144°C |
| Cl | $-C-NH_2$<br>‖<br>S | H | 217 (decomp.) |

The compounds of the present invention are useful in the inhibition of HIV reverse transcriptase, the prevention or treatment of infection by the human immunodeficiency virus (HIV) and the treatment of consequent pathological conditions such as AIDS. Treating AIDS or preventing or treating infection by HIV is defined as including, but not limited to, treating a wide range of states of HIV infection: AIDS, ARC (AIDS related complex), both symptomatic and asymptomatic, and actual or potential exposure to HIV. For example, the compounds of this invention are useful in treating infection by HIV after suspected past exposure to HIV by, e.g., blood transfusion, organ transplant, exchange of body fluids, bites, accidental needle stick, or exposure to patient blood during surgery.

For these purposes, the compounds of the present invention may be administered orally, parenterally (including subcutaneous injections, intravenous, intramuscular, intrasternal injection or infusion techniques), by inhalation spray, or rectally, in dosage unit formulations containing conventional non-toxic pharmaceutically-acceptable carriers, adjuvants and vehicles.

Thus, in accordance with the present invention there is further provided a method of treating and a pharmaceutical composition for treating HIV infection and AIDS. The treatment involves administering to a

patient in need of such treatment a pharmaceutical composition comprising a pharmaceutical carrier and a therapeutically-effective amount of a compound of the present invention.

These pharmaceutical compositions may be in the form of orally-administrable suspensions or tablets; nasal sprays; sterile injectable preparations, for example, as sterile injectable aqueous or oleagenous suspensions or suppositories.

When administered orally as a suspension, these compositions are prepared according to techniques well-known in the art of pharmaceutical formulation and may contain microcrystalline cellulose for imparting bulk, alginic acid or sodium alginate as a suspending agent, methylcellulose as a viscosity enhancer, and sweetners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these compositions may be prepared by mixing the drug with a suitable non-irritating excipient, such as cocoa butter, synthetic glyceride esters or polyethylene glycols, which are solid at ordinary temperatures, but liquidify and/or dissolve in the rectal cavity to release the drug.

The compounds of this invention can be administered orally to humans in a dosage range of 1 to 100 mg/kg body weight in divided doses. One preferred dosage range is 1 to 10 mg/kg body weight orally in divided doses. Another preferred dosage range is 1 to 20 mg/kg body weight orally in divided doses. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The present invention is also directed to combinations of the HIV reverse transcriptase inhibitor compounds with one or more agents useful in the treatment of AIDS. For example, the compounds of this invention may be effectively administered, whether at periods of pre-exposure and/or post-exposure, in combination with effective amounts of the AIDS antivirals, immunomodulators, anti-infectives, or vaccines known to those of ordinary skill in the art.

It will be understood that the scope of combinations of the compounds of this invention with AIDS antivirals, immunomodulators, anti-infectives or vaccines include in principle any combination with any pharmaceutical composition useful for the treatment of AIDS.

## REVERSE TRANSCRIPTASE ASSAY

The assay measures the incorporation of tritiated deoxyguanosine monophosphate by recombinant HIV reverse transcriptase (HIV $RT_R$) (or other RT) into acid-precipitable cDNA at the Km values of dGTP and poly r(C)•oligo $d(G)_{12-18}$. The inhibitors of the present invention inhibit this incorporation.

Thirty uL of a reaction mixture containing equal volumes of: 500 mM Tris•HCl (pH 8.2), 300 mM $MgCl_2$, 1200 mM KCl, 10 mM DTT, 400 $\mu$g/mL poly r(c)•oligo d(G) [prepared by dissolving 1.5 mg (25 U) poly r-(C)•oligo d(G) in 1.5 ml sterile distilled $H_2O$ and diluting to 400 $\mu$g/ml], 0.1 $\mu$Ci/$\mu$l [$^3$H] dGTP, 160 $\mu$M dGTP, was added to 10 $\mu$l sterile distilled $H_2O$, 2.5 $\mu$l of potential inhibitor and 10 $\mu$L of 5 nM purified HIV $RT_R$ in tubes. The mixture was incubated at 37°C for 45 minutes.

After incubation is complete, the tubes were cooled in ice for 5 minutes. Ice-cold 13% TCA containing 10 mM $NaPP_i$ (200 $\mu$l) are added and the mixture incubated on ice for 30 minutes. The precipitated cDNA is removed by filtration using presoaked glass filters [TCA, $NaPP_i$]. The precipitate is then washed with 1N HCl, 10 mM $NaPP_i$.

The filter discs are then counted in a scintillation counter.

Under these conditions [dGTP] and poly r(C)•oligo $d(G)_{12-18}$ each are approximately equal to the appropriate Km value. Approximately 5-6,000 cpm of [$^3$H] dGMP are incorporated into acid-precipitable material. The RT reaction is concentration- and time-dependent. DMSO (up to 5%) does not affect enzyme

activity. Calculated $IC_{50}$ values for the tested compounds of this invention vary from about 6 nM to more than 300 μm. The $IC_{50}$ values of the most preferred compounds range from about 6 nM to about 30 nM.

INHIBITION OF VIRUS SPREAD

A. Preparation of HIV-infected MT-4 Cell Suspension.

MT cells were infected at Day O at a concentration of 250,000 per ml with a 1:2000 dilution of HIV-1 strain IIIb stock (final 125 pg p24/ml; sufficient to yield ≦ 1% infected cells on day 1 and 25-100% on day 4). Cells were infected and grown in the following medium: RPMI 1640 (Whittaker BioProducts), 10% inactivated fetal bovine serum, 4 mM glutamine (Gibco Labs) and 1:100 Penicillin-Streptomycin (Gibco Labs).

The mixture was incubated overnight at 37°C in 5% $CO_2$ atmosphere.

B. Treatment with Inhibitors

Serial two-fold dilutions of compound were prepared in cell culture medium. At Day 1, aliquots of 125 μl of compound were added to equal volumes of HIV-infected MT-4 cells (50,000 per well) in a 96-well microtiter cell culture plate. Incubation was continued for 3 days at 37°C in 5% $CO_2$ atmosphere.

C. Measurement of Virus Spread

Using a multichannel pipettor, the settled cells were resuspended and a 125 μl harvested into a separate microtiter plate. After the settling of the cells, the plates were frozen for subsequent assay of the supernatant for HIV p24 antigen.

The concentration of HIV p24 antigen was measured by an enzyme immunoassay, described as follows. Aliquots of p24 antigen to be measured were added to microwells coated with a monoclonal antibody specific for HIV core antigen. The microwells were washed at this point, and at other appropriate steps that follow. Biotinylated HIV-specific antibody was then added, followed by conjugated streptavidin-horseradish peroxidase. A color reaction occurs from the added hydrogen peroxide and tetramethylbenzidine substrate. Color intensity is proportional to the concentration of HIV p24 antigen.

The cell culture inhibitory concentration ($CIC_{95}$) for each compound is defined as that concentration which inhibited by greater than 95% the spread of infection, as assessed by a greater than 95% reduction in p24 antigen production relative to untreated controls. The tested compounds of the present invention were found to have $CIC_{95}$ values ranging from about 3 nM to about 400 nM for preferred species, and up to about 40 μM for others.

**EXAMPLE 1**

**Preparation of N-(3-Pyridylmethyl)-5-chloro-3-phenylthioindole-2-carboxamide**

Step A: 5-Chloro-3-phenylthioindole-2-carboxylic acid

To a suspension of sodium hydride ( 3.0 g, 60% dispersion in oil, 0.076 mol) in dimethylformamide (125 mL) was added 5-chloroindole-2-carboxylic acid (5.0 g, 0.0255 mol) and phenyldisulfide (6.1 g, 0.028 mol). The reaction was heated under nitrogen at 50°C overnight. The reaction was cooled, and additional sodium hydride (1.8 g) and phenyldisulfide (3.6 g) were added and heating continued for 1 h. The reaction was cooled and the dimethylformamide distilled in vacuo. The residue was partitioned between ethyl acetate and water. The aqueous layer was separated and the pH adjusted to pH1 with 10% aqueous hydrochloric acid. The aqueous phase was extracted with ethyl acetate, and the ethyl acetate extract was washed with water and saturated brine, and dried over magnesium sulfate. The crude product was recrystallized from ethyl acetate in hexane to afford the title compound as an off-white solid.

Step B: N-(3-Pyridylmethyl)-5-chloro-3-phenylthioindole-2-carboxamide

Benzotriazol-1-yloxytris(dimethylamino)phosphonium hexafluorphosphate (0.73 g, 1.6 mmol) was added to a solution of 5-chloro-3-phenylthioindole-2-carboxylic acid (0.50 g, 1.6 mmol), 3-aminomethylpyridine (0.35 g, 3.2 mmol) and triethylamine (0.50 mL, 3.2 mmol) in degassed dimethylformamide (25 mL). The

reaction was stirred at room temperature overnight. The precipitated product was filtered and the filter cake washed well with water. The solid was triturated with 30% ethyl acetate in hexane, filtered and dried at 60°C in vacuo for 72 h. The title compound was obtained as an off-white solid, mp 240-241 °C. Anal. Calc. for $C_{21}H_{16}ClN_3OS \cdot 0.25\ H_20$: C, 63.31; H, 4.17; N, 10.54. Found: C, 63.34; H, 4.06; N, 10.71. NMR (DMSO-$d_6$): δ 12.54 (1H, s), 8.91 (1H, t, J=6 Hz), 8.51 (1H, s), 8.42 (1H, d, J=5 Hz), 7.58 (2H, m), 7.45 (1H, m), 7.25 (4H, m), 7.15 (1H, t, J=7 Hz), 7.04 (2H, d, J=8 Hz), 4.58 (2H, d, J=6 Hz).

## EXAMPLE 2

### Preparation of Methyl 5-chloro-3-phenylthioindole-2-carboxylate

Oxalyl chloride (0.70 mL, 9.6 mmol) was added to a solution of 5-chloro-3-phenylthioindole-2-carboxylic acid (0.97 g, 3.2 mmol) in chloroform (50 mL) under nitrogen. The reaction was refluxed for 3 h, cooled and reduced to dryness in vacuo. The resulting solid was dissolved in chloroform and added to methanol at 0 °C. The methanol was removed in vacuo and the crude product chromatographed on silica gel with 20% ethyl acetate in hexane. The title compound was obtained as a solid, mp 193-196 °C. Anal. Calc. for $C_{16}H_{12}ClNO_2S$: C, 60.47; H, 3.81; N, 4.42. Found: C, 60.09; H, 3.50; N, 4.67.

## EXAMPLE 3

### Preparation of Ethyl 5-chloro-3-benzylindole-2-carboxylate

The title compound was prepared according to the procedure described by Inaba, S., et al., Chem. Pharm. Bull., 24, 1076-1082 (1976). Recrystallization from benzene gave the title compound as pale yellow needles, mp196-197 °C. Anal. Calc. for $C_{18}H_{16}ClNO_2$: C, 68.90; H, 5.13; N, 4.46. Found: C, 68.64; H, 5.10; N, 4.56.

## EXAMPLE 4

### Preparation of 2-Phenylsulfinylmethyl-3-phenylthioindole

Step A: 2-Hydroxymethylindole

A suspension of lithium aluminum hydride (2.0 g, 0.20 mol) in tetrahydrofuran (100 mL) was cooled with stirring to 0 °C under nitrogen. A solution of ethyl indole-2-carboxylate (10.0 g, 0.052 mol) in tetrahydrofuran was added dropwise, maintaining the reaction temperature between 0-5 °C. After 1 h, the reaction was quenched with saturated sodium potassium tartrate solution. The reaction was filtered and the filter cake washed well with tetrahydrofuran. The tetrahydrofuran was evaporated in vacuo and the residue partitioned between ethyl acetate and water. The ethyl acetate solution was washed with water, saturated brine, dried over magnesium sulfate, filtered and freed of solvent. The title compound was obtained as a yellowish solid. NMR ($CDCl_3$): δ 8.18 (1H, bs), 7.57 (1H, d, J=8 Hz), 7.35 (1H, d, J=8 Hz), 7.26 (1H, s), 7.18 (1H, dt, J=1, 8 Hz), 7.10 (1H, dt, J=1, 8 Hz), 6.41 (1H, bs), 4.84 (2H, s).

Step B: 2-Phenylthiomethylindole

2-Hydroxymethylindole (6.94 g, 0.047 mol) and phenyldisulfide (10.8 g, 0.049 mol) were dissolved in tetrahydrofuran (200 mL) and cooled to 0 °C under nitrogen. Tri-n-butylphosphine (11.7 mL, 0.047 mol) was added and the reaction stirred for 1 h. Additional phenyldisulfide (1.5 g, 0.007) and tri-n-butylphosphine (5.1 mL, 0.20 mol) was added, and the reaction stirred at room temperature until complete. The tetrahydrofuran was removed in vacuo and the residue chromatographed on silica gel eluting with 5% ethyl acetate in hexane. The title compound was obtained as clear colorless plates, mp 100-101.5 °C. Anal. Calc. for $C_{15}H_{13}NS$: C, 75.27, H, 5.47, N, 5.85. Found: C, 74.52, H, 5.39, N, 5.95.

Step C: 3-Phenylthio-2-phenylthiomethylindole

A suspension of sodium hydride (0.37 g 60% dispersion in oil, 9.4 mmol) in dimethylformamide (50 mL) was cooled to 0 °C. 2-Phenylthiomethylindole (1.5 g, 6.3 mmol) was added portionwise, and the reaction stirred at 0 °C for 15 min. Phenyldisulfide (1.5 g, 6.9 mmol) was added and the reaction stirred at 20 °C for

6 h. The reaction was quenched with water and extracted with ethyl acetate. The organic extract was washed with water, saturated brine and dried over magnesium sulfate. Filtration and evaporation of solvent left an oil which was purified by medium pressure chromatography on silica gel using 5% ethyl acetate in hexane. The title compound was obtained as an oil. Anal. Calc. for $C_{21}H_{17}NS_2 \cdot H_2O \cdot 0.15\ C_4H_8O_2$: C, 68.50; H, 5.33; N 3.60. Found: C, 68.40; H, 4.65; N, 3.86.

Step D: 2-Phenylsulfinylmethyl-3-phenylthioindole

A solution of 3-phenylthio-2-phenylthiomethylindole (0.750 g, 2.94 mmol) in methanol (100 mL) was cooled to 0 °C with stirring. Monoperoxyphthalic acid magnesium salt (0.908 g, 80% peracid) in methanol (50 mL) was added slowly dropwise. After addition, the reaction was stirred an additional 30 min., then quenched with 10% aqueous sodium thiosulfate (2 mL). The methanol was removed in vacuo, and the residue partitioned between ethyl acetate and water. The organic phase was washed successively with water and saturated brine, then dried over magnesium sulfate. Filtration and concentration of the filtrate in vacuo gave an oil which was purified by chromatography on silica gel using 20-30% ethyl acetate in hexane. The title compound was obtained as a foam, mp 71-74 °C. Exact mass calculated for $C_{21}H_{17}NOS_2$: 364.082982. Found: 364.084549. NMR (DMSO-$d_6$) $\delta$ 11.82 (1H, s), 7.50 (6H, m), 7.23 (1H, d, J = 8 Hz), 7.15 (3 H, m) 7.05 (2H, m) 6.90 2H, m), 4.43 (1H, d, J = 13 Hz), 4.38 (1H, d, J = 13 Hz).

## EXAMPLE 5

### Preparation of 2-Phenylcarboxamidomethyl-3-phenylthioindole

Step A: 3-Phenythioindole-2-carboxamide

The title compound was prepared from 3-phenylthioindole-2-carboxylic acid (prepared according to the procedure described by Atkinson, J.G. et al. , Synthesis, p. 480-481 (1988), (4.01 g, 0.015 mol), ammonia (large excess), and benzotriazol-1-yloxytris(dimethylamino)phosphonium hexafluorphosphate (7.2 g, 0.016 mol) in dimethylformamide according to the general procedure described in Example 1 for the preparation of N-(3-pyridylmethyl)-5-chloro-3-phenylthio-2-carboxamide. The title compound was obtained as a pale yellow solid.

Step B: 2-Aminomethyl-3-phenylthioindole

A solution of 3-phenylthioindole-2-carboxamide (1.9 g, 7.1 mmol) in tetrahydrofuran was cooled under nitrogen to 0 °C and treated with neat borane-dimethylsulfide complex (7.1 mL, 0.070 mol). The reaction was refluxed for 7 h, cooled to 0 °C and quenched with 10% aqueous hydrochloric acid. The solution was adjusted to pH 8 with 20% aqueous sodium hydroxide. The reaction was extracted with ethyl acetate and the organic extract washed with saturated brine, and dried over magnesium sulfate. The title compound was obtained as a pale yellow oil.

Step C: 2-Phenylcarboxamidomethyl-3-phenylthioindole

2-Aminomethyl-3-phenylthioindole (0.85 g, 3.3 mmol) was dissolved in chloroform (15 mL) and cooled under nitrogen to 0ºC. Pyridine (2.7 mL, 33 mmol) was added, followed by benzoyl chloride (1.1 mL, 10 mmol). The reaction was stirred at 20 °C for 1 h and 10% aqueous hydrochloric acid added. The layers were separated and the organic phase washed successively with water, saturated sodium bicarbonate and saturated brine. The chloroform solution was dried over magnesium sulfate, filtered and evaporated to dryness. The resulting oil was chromatographed on silica gel with 5% ethyl acetate in methylene chloride. The title compound was obtained as a solid, mp 64-65 °C. Anal. Calc. for $C_{22}H_{18}N_2OS \cdot 0.2\ H_2O$: C, 73.00; H, 5.08; N, 7.74. Found: C, 72.93; H, 5.02; N, 7.66.

**EXAMPLE 6**

**Preparation of 2-(N-Phenylacetamido)-3-phenylthioindole and 2-(N-Phenylacetamido)-1-(phenylcarbamoyl)-3-phenylthioindole**

2-Methyl-3-phenylthioindole (0.50 g, 2.1 mmol) (prepared according to the procedure described by Atkinson, J. G., et al., Synthesis, p. 480-481 (1988), was dissolved in dry tetrahydrofuran and cooled under nitrogen to -78°C. A solution of n-butyllithium in hexane (0.83 mL, 2.5 M) was added via syringe. Carbon dioxide was bubbled into the reaction mixture over a period of several minutes; unreacted carbon dioxide was removed by freezing the reaction at liquid nitrogen temperature under high vacuum and warming to -78°C. A solution of t-butyllithium in hexane was added (1.35 mL, 1.7 M) and the reaction stirred for 20 min. Phenylisocyanate (0.23 mL, 2.1 mmol) in tetrahydrofuran (1.5 mL) was added and the reaction stirred at 20°C overnight. The reaction was diluted with water and extracted with ethyl acetate. The organic phase was washed with saturated brine and dried over magnesium sulfate. Filtration and evaporation of solvent left an amber oil. The crude products were chromatographed on silica gel eluting successively with 15%, 20%, and 40% ether in hexane. 2-(N-Phenylacetamido)-3-phenylthioindole was isolated as a solid, mp 66-68°C. Anal. Calc. for $C_{22}H_{18}N_2OS$: C, 72.98; H, 5.01; N, 7.73. Found: C, 72.99; H, 4.87; N, 7.52. Later fractions contained 2-(N-phenylacetamido)-1-(phenylcarbamoyl)-3-phenylthioindole, mp 123-125°C. Anal. Calc. for $C_{29}H_{23}N_3O_2S$: C, 70.28, H, 4.67, N, 8.47. Found: C, 70.37, H, 4.61; N, 8.34.

**EXAMPLE 7**

**Preparation of 2-(2-Oxo-2-furan-3-yl)ethyl-3-phenylthioindole**

Step A: N-Methoxy-N-methylfuran-3-carboxamide

The title compound was prepared from furan-3-carboxylic acid (3.4 g, 0.030 mol), N,O-dimethylhydroxylamine hydrochloride hydrochloride (2.9 g, 0.030 mol) triethylamine (8.3 mL, 0.060 mol) and benzotriazol-1-yloxytris(dimethylamino)phosphonium hexafluorphosphate (13.3 g, 0.030 mol) according to the general procedure described in Example 1 for N-(3-pyridylmethyl)-5-chloro-3-phenylthio-2-carboxamide. NMR (DMSO-$d_6$) δ 8.25 (1H, s), 7.75 (1H, s), 3.70 (3H, s), 3.22 (3H, s).

Step B: 2-(2-Oxo-2-furan-3-yl)ethyl-3-phenylthioindole

The title compound was prepared from N-methoxy-N-methylfuran-3-carboxamide (0.32 g, 2.1 mmol), and 2-methyl-3-phenylthioindole (0.50 g, 2.1 mmol) according to the general procedure described in Example 6 for the preparation of 2-(N-phenylacetamido)-3-phenyl-thioindole.The crude product was chromatographed on silica gel with chloroform. The title compound was obtained as a pale yellow solid, mp 127-129°C. Anal. Calc. for $C_{20}H_{15}NO_2S$: C, 72.05; H, 4.54; N, 4.20. Found: C, 72.08; H, 4.57; N, 4.24.

**EXAMPLE 8**

**Preparation of 2-Benzoyl-5-chloro-3-phenylthioindole**

Step A: N-Methoxy-N-methyl-5-chloro-3-phenylthioindole-2-carboxamide

The title compound was prepared from 5-chloro-3-phenylthioindole-2-carboxylic acid (1.0 g, 3.30 mmol)) N,O-dimethylhydroxylamine hydrochloride (0.64 g, 6.6 mmol), triethylamine (1.0 mL, 7 mmol) and benzotriazol-1-yloxytris(dimethylamino)phosphonium hexafluorphosphate (1.64 g, 3.6 mmol) in dimethylformamide according to the general procedure described in Example 1 for the preparation of N-(3-pyridylmethyl)-5-chloro-3-phenylthio-2-carboxamide.

Step B: 2-Benzoyl-5-chloro-3-phenylthioindole

N-Methoxy-N-methyl-5-chloro-3-phenylthioindole-2-carboxamide (0.24 g, 0.69 mmol) was dissolved in dry tetrahydrofuran (5 mL) and cooled to -78°C under nitrogen. A solution of phenylmagnesium chloride in tetrahydrofuran (0.81 mL, 2M) was added via syringe and the reaction warmed to 20°C overnight. Water and ethyl acetate were added to the reaction and then separated. The organic phase was washed with

water, 5% aqueous hydrochloric acid, saturated sodium bicarbonate, saturated brine, and dried over magnesium sulfate. Filtration and evaporation of solvent gave the crude product which was chromatographed on silica gel with 10% ether in hexane. The title compound was obtained as a solid, mp 154-155 °C. Anal. calc. for $C_{21}H_{14}ClNOS$: C, 69.32; H, 3.88; N, 3.85. Found: C, 68.61; H, 3.83; N, 3.83.

**EXAMPLE 9**

**Preparation of 2-(2-Benzoxazol-2-ylethyl)-3-phenylthioindole**

Step A: N-Methoxy-N-methyl-3-phenylthioindole-2-carboxamide

The title compound was prepared from 3-phenylthioindole-2-carboxylic acid (1.0 g, 3.7 mmol), N,O-dimethylhydroxylamine hydrochloride (0.54 g, 5.5 mmol), triethylamine (1.5 mL, 11 mmol) and benzotriazol-1-yloxytris(dimethylamino)phosphonium hexafluorphosphate (1.64 g, 3.7 mmol) according to the procedure described in Example 1 for N-(3-pyridylmethyl)-5-chloro-3-phenylthio-2-carboxamide.

Step B: 3-Phenylthioindole-2-carboxaldehyde

N-Methoxy-N-methyl-3-phenylthioindole-2-carboxamide (1.57 g, 5.26 mmol) was dissolved in tetrahydrofuran (150 mL) and cooled to 0 °C under nitrogen. A solution of lithium aluminum hydride in tetrahydrofuran (5.76 mL, 1M) was added slowly via syringe and the reaction stirred a total of 1.5 h. Ethyl acetate (30 mL) was added, followed by saturated sodium potassium tartrate solution. The layers were separated and the organic phase washed with saturated brine and dried over magnesium sulfate. Filtration and evaporation of solvent gave the title compound as a yellow solid.

Step C: trans-2-(2-Benzoxazol-2-ylethenyl)-3-phenylthioindole

n-Butyllithium in hexane (3.47 mL, 2.5 M) was added to a solution of [(benzoxal-2-yl)methyl]-diethylphosphonate (2.34 g, 8.68 mmol) in tetrahydrofuran (50 mL) at -78oC under nitrogen. The reaction was stirred for 20 min., and warmed to -20 °C. A solution of 3-phenylthioindole-2-carboxaldehyde (1.10 g, 4.34 mmol) in tetrahydrofuran (30 mL) was added and the reaction stirred at 20 °C overnight. Ethyl acetate and water were added and the layers separated. The organic layer was washed with saturated brine and dried over magnesium sulfate. The crude product was triturated with 1:1 hexane ethyl acetate and collected by filtration. The title compound was obtained as a yellow solid, mp 260 °C. Anal. Calc. for $C_{23}H_{16}N_2OS$: C, 72,32; H, 4.58; N, 7.33. Found: C, 72.41; H, 4.50; N, 7.44.

Step D: 2-(2-Benzoxazol-2-ylethyl)-3-phenylthioindole

A solution of trans-2-(2-benzoxazol-2-ylethenyl)-3-phenylthioindole (0.420 g, 1.14 mmol) in 1:1 methanol/tetrahydrofuran (250 mL) was stirred under 1 atmosphere of hydrogen in the presence of 10% palladium on charcoal (100 mg). Additional catalyst was added as needed to drive the reaction to completion. The catalyst was removed by filtration, and the filtrate concentrated in vacuo. The resulting solid was triturated with 10% ethyl acetate in hexane and collected by filtration to afford the title compound, mp 192-193 °C. Anal. calc. for $C_{23}H_{18}N_2OS$: C, 72.80; H, 5.04; N, 7.38. Found: C, 72.78; H, 4.95; N, 7.45.

**EXAMPLE 10**

**Preparation of N-2-Furanylmethyl-5-chloro-3-phenylthioindole-2-carboxamide**

The title compound was prepared according to the procedure described in example 1, step B, except substituting 2-aminomethylfuran for 3-aminomethylpyridine. The dimethylformamide was removed in vacuo, and the residue triturated with 1:1 ethyl acetate-hexane and filtered. Recrystallization from acetonitrile gave the title compound, mp 214 °C. Anal. Calc. for $C_{20}H_{15}ClN_2O_2S$: C. 62.74; H, 3.95; N 7.32. Found: C, 62.27; H, 3.88; N, 7.41.
NMR (DMSO-$d_6$): $\delta$ 12.55 (1H, s), 8.72 (1H, t, J=6 Hz), 7.55 (1H, m), 7.54 (1H, d, J=8 Hz), 7.45 (1H, d, J=2 Hz), 7.15 (1H, tt, J=7, 1 Hz), 7.08 (1H, s), 7.06 (1H, d, 3= 8 Hz), 6.35 (1H, m), 6.18 (1H, m), 4.56 (2H, d, J=6 Hz).

**EXAMPLE 11**

**Preparation of N-3-Pyridyl-5-chloro-3-phenylthioindole-2-carboxamide**

The title compound was prepared according to the procedure described in example 1, step B, except substituting 3-aminopyridine for 3-aminomethylpyridine. The dimethylformamide was removed in vacuo, and the residue triturated with 1:1 ethyl acetate-hexane and filtered. Chromatography on silica gel with 40% ethyl acetate in hexane gave the title compound, mp 255-256°C. Anal. Calc. for $C_{20}H_{15}ClN_3OS$: C, 63.24; H, 3.98; N, 10.74. Found: C, 62.59; H, 3.86; N, 11.06. NMR (DMSO-$d_6$): δ 12.72 (1H, s), 10.55 (1H, s), 8.85 (1H, d, J=3 Hz), 8.35 (1H, dd, J=5, 1 Hz), 8.14 (1H, dm, $J_d$=8 Hz), 7.60 (1H, d, J=9 Hz), 7.48 (1H, d, J=2 Hz), 7.40 (1H, dd, J=9, 2 Hz), 7.25 (2H, t, J=7 Hz), 7.16 (1H, m), 7.11 (2H, t, J=7 Hz).

**EXAMPLE 12**

**Preparation of N-Ethyl-5-chloro-3-phenylthioindole-2-carboxamide**

The title compound was prepared according to the procedure described in example 1, step B, except substituting ethylamine for 3-aminomethylpyridine. The dimethylformamide was removed in vacuo, and the residue triturated with 1:1 ethyl acetate-hexane and filtered. Recrystallization from 2% methanol in ethyl acetate gave the title compound, mp 210-211°C. Anal. Calc. for $C_{17}H_{15}ClN_2OS$ • 0.5 $H_2O$: C, 60.08; H, 4.74; N, 8.24. Found: C, 60.00; H, 4.18; N, 8.52. NMR (DMSO-$d_6$): δ 12.49 (1H, s), 8.31(1H, t, J=6 Hz), 7.54 (1H, d, J=9 Hz), 7.43 (1H, d, J= 2 Hz), 7.27 (3H, m), 7.15 (1H, tt, J=7, 2 Hz), 7.07 (2H, m), 3.35(4H, m), 1.06 (3H, t, J=7 Hz).

**EXAMPLE 13**

**Preparation of N-3-Methoxybenzyl-5-chloro-3-phenylthioindole-2-carboxamide**

The title compound was prepared according to the procedure described in example 1, step B, except substituting 3-methoxybenzylamine for 3-aminomethylpyridine. The dimethylformamide was removed in vacuo, and the residue triturated with 1:1 ethyl acetate-hexane and filtered. Recrystallization from acetonitrile gave the title compound, mp 172°C. Anal. Calc. for $C_{23}H_{19}ClN_2O_2S$ • 0.3 $H_2O$: C, 64.48; H, 4.61; N, 6.54. Found: C, 64.41; H, 4.38; N, 6.75. NMR (DMSO-$d_6$): δ 12.55 (1H, s), 8.80 (1H, m), 7.55 (1H, d, J=8 Hz), 7.44 (1H, s), 7.25 (3H, m), 7.15 (2H, m), 7.05 (2H, d, J=7 Hz), 6.80 (3H, m), 4.54 (2H, d, J=6 Hz).

**EXAMPLE 14**

**Preparation of N-2-Methoxyethyl-5-chloro-3-phenylthioindole-2-carboxamide**

The title compound was prepared according to the procedure described in example 1, step B, except substituting 2-methoxyethylamine for 3-aminomethylpyridine. The dimethylformamide was removed in vacuo, and the residue triturated with 1:1 ethyl acetate-hexane and filtered to give the title compound, mp 216-217°C. Anal. Calc. for $C_{18}H_{17}ClN_2O_2S$: 0.25 $H_2O$: C, 59.17; H, 4.83; N, 7.67. Found: C, 59.11; H, 4.75; N, 7.82. NMR (DMSO-$d_6$): δ 12.54 (1H, s), 8.44 (1H, t, J=6 Hz), 7.54 (1H, d, J=9 Hz), 7.48 (1H, d, J=2 Hz), 7.28 (3H, m), 7.17 (1H, t, J=7 Hz), 7.10 (2H, m), 3.49 (2H, q, J=6 Hz), 3.37 (2H, t, J=6 Hz), 3.16 (3H, s).

**EXAMPLE 15**

**Preparation of N-4-Pyridylmethyl-5-chloro-3-phenylthioindole-2-carboxamide**

The title compound was prepared according to the procedure described in example 1, step B, except substituting 4-aminomethylpyridine for 3-aminomethylpyridine. The dimethylformamide was removed in vacuo, and the residue triturated with 1:1 ethyl acetate-hexane and filtered. Recrystallization from acetonitrile gave the title compound, mp 228-229°C. Anal. Calc. for $C_{21}H_{16}ClN_3OS$: 0.2 $H_2O$: C, 63.45; H, 4.16; N, 10.57. Found: C, 63.33; H, 4.02; N, 10.50. NMR (DMSO-$d_6$): δ 12.56 (1H, s), 8.92 (1H, t, J= 6 Hz), 8.38 (1H, d, J=4 Hz), 7.55 (1H, d, J=8 Hz), 7.47 (1H, s), 7.31 (1H, dd, J=8, 2 Hz), 7.25 (1H, d, J=7 Hz), 7.17 (2H, m), 7.05 (1H, d, J=7 Hz), 4.58 (2H, d, J=6 Hz).

## EXAMPLE 16

### Preparation of N-2-Hydroxyethyl-5-chloro-3-phenylthioindole-2-carboxamide

The title compound was prepared according to the procedure described in example 1, step B, except substituting 2-hydroxyethylamine for 3-aminomethylpyridine. The dimethylformamide was removed in vacuo, and the residue triturated with 1:1 ethyl acetate-hexane and filtered. Chromatography on silica gel with 2% methanol in chloroform gave the title compound, mp 222-223 °C. Anal. Calc. for $C_{17}H_{15}ClN_2O_2S$: 0.3 $H_2O$: C, 57.96; H, 4.46; N, 7.95. Found: C, 57.99; H, 4.26; N, 7.90. NMR (DMSO-$d_6$): $\delta$ 12.50 (1H, s), 8.46 (1H, m), 7.55 (1H, d, J=9 Hz), 7.45 (1H, d, J=1 Hz), 7.28 (3H, m), 7.17 (1H, t, J=6 Hz), 7.13 (2H, m), 4.85 (1H, t), 3.49 (1H, m), 3.43 (1H, m).

## EXAMPLE 17

### Preparation of 5-Chloro-3-phenylthioindole-2-carboxamide

The title compound was prepared according to the procedure described in example 1, step B, except substituting an excess of ammonia gas for 3-aminomethylpyridine and triethylamine. The dimethylformamide and excess ammonia were removed in vacuo and the residue partitioned between ethyl acetate and 10% hydrochloric acid. The organic phase was washed with water, 5% sodium hydroxide and saturated brine, and then dried over magnesium sulfate. Filtration and evaporation gave a crude product which was chromatographed on silica gel with 30% ethyl acetate in hexane. The title compound was obtained as a white solid mp 213-215 °C. Anal. Calc. for $C_{15}H_{11}ClN_2OS \cdot 1/3H_2O$: C,58.35; H, 3.81; N, 9.07. Found. C, 58.33; H, 3.64; N, 9.11. NMR (DMSO-$d_6$): $\delta$ 12.52 (1H, bs), 8.06 (1H, s), 7.76 (1H, s), 7.55 (1H, d, J=9 Hz), 7.44 (1H, s), 7.28 (3H, m), 7.15 (1H, t, J=6 Hz), 7.06 (2H, d, J=8 Hz).

## EXAMPLE 18

### Preparation of 5-Chloro-3-phenylthioindole-2-thiocarboxamide

A solution of 5-chloro-3-phenylthioindole-2-carboxamide (3.8 g, 12.5 mmol) and [2,4-bis(4-methoxyphenyl)-1,3-dithia-2,4-diphosphetane-2,4-disulfide (Lawesson's reagent) (5.0 g, 12.5 mmol) in dry THF (110 mL) was refluxed under nitrogen for 16 h. The solvent was removed in vacuo and the residue chromatographed on silica gel with 10% ethyl acetate in hexane. The chromatographed product was triturated with hexane and the yellowish solid collected and dried to give the title compound, mp 217 °C (decomposed). Anal. Calc. for $C_{15}H_{11}ClN_2S$: C, 56.50; H, 3.48; N, 8.79. Found: C, 56.75; H, 3.64; N, 8.59. NMR (DMSO-$d_6$): $\delta$ 12.22 (1H, s), 10.31 (1H, s), 9.48 (1H, s), 7.50 (1H, d, J=8 Hz), 7.39 (1H, s), 7.25 (3H, m), 7.13 (1H, t, J=7 Hz), 7.01 (2H, d, J=7 Hz).

## EXAMPLE 19

### Preparation of N-2-furanylmethyl-5-chloro-3-phenylthioindole-2-thiocarboxamide

The title compound was prepared according to the procedure described for 5-chloro-3-phenylthioindole-2-thiocarboxamide except substituting N-2-furanylmethyl-5-chloro-3-phenylthioindole-2-carboxamide for 5-chloro-3-phenylthioindole-2-carboxamide. The crude product was chromatographed on silica gel with 3% ethyl acetate in hexane. The title compound was obtained as a bright yellow solid, mp 143-144 °C. Anal. Calc. for $C_{20}H_{15}ClN_2OS_2 \cdot H_2O$: C, 57.61; H, 3.62; N, 6.72. Found: C, 57.56; H, 3.58; N, 6.52. NMR (DMSO-$d_6$): $\delta$ 12.27 (1H, s), 10.73 (1H, s), 7.55 (2H, m), 7.39 (1H, s), 7.21 (4H, m), 6.98 (2H, d, J=7 Hz), 6.36 (2H, 4.96 (2H, s).

## EXAMPLE 20

### Preparation of N-[1-(2(R)-hydroxypropyl)]-5-chloro-3-phenylthioindole-2-carboxamide

The title compound was prepared according to the procedure described in example 1, step B, except substituting 2(R)-hydroxy-1-propylamine for 3-aminomethylpyridine. The dimethylformamide was removed in vacuo and the residue triturated first with 20% ethyl acetate in hexane then by acetonitrile. The title

compound was obtained as an off-white solid, mp 202-203 °C. Anal. Calc. for $C_{18}H_{17}ClN_2O_2S \cdot 0.3\ H_2O$: C, 59.01; H, 4.67; N, 7.65. Found: C, 58.91; H, 4.59; N, 7.50. NMR (DMSO-$d_6$): δ 12.52 (1H, s), 8.45 (1H, t, J=5 Hz), 7.55 (1H, d, J=8 Hz), 7.46 (1H, s), 7.25 (3H, m), 7.15 (3H, m), 4.89 (1H, d, J=5 Hz), 3.74 (1H, m), 3.38 (1H, m), 3.23 (1H, m), 1.00 (3H, d, J=6 Hz).

**EXAMPLE 21**

**Preparation of N-(2-pyridyl)methyl-5-chloro-3-phenylthioindole-2-carboxamide**

The title compound was prepared according to the procedure described in example 1, step B, except substituting 2-pyridylmethylamine for 3-aminomethylpyridine. The dimethylformamide was removed in vacuo and the residue triturated first with 30% ethyl acetate in hexane, then with acetonitrile. The title compound was obtained as a white solid, mp 209-210 °C. Anal. Calc. for $C_{21}H_{16}ClN_3OS$: C, 64.03; H, 4.10; N, 10.67. Found: C, 63.51; H, 3.97; N, 10.41. NMR (DMSO-$d_6$): δ 12.58 (1H, s), 9.15 (1H, t, J=5 Hz), 8.46 (1H, d, J=5 Hz), 7.66 (1H, t, J=8 Hz), 7.57 (1H, d, J=8 Hz), 7.50 (1H, s), 7.25 (5H, m), 7.12 (3H, m), 4.68 (2H, d, J=5 Hz).

**EXAMPLE 22**

**Preparation of N-(3-methoxy-4-pyridyl)methyl-5-chloro-3-phenylthioindole-2-carboxamide**

Step 1: Preparation of 4-cyano-2-methoxypyridine

A solution of 2-chloro-4-cyanopyridine (1.25 g, 9.1 mmol), prepared as described by D. Libermann, N. Rist, F. Grumbach, S. Cals, M. Moyeux and A. Rouaix, Bull. Soc. Chim. France, 694 (1958), in methanol was treated with sodium methoxide (0.58 g, 10.9 mmol) and refluxed for 30 minutes. The reaction mixture was cooled, filtered and the filtrate concentrated in vacuo to obtain the crude product as an off-white solid. The crude product was chromatographed on silica gel with 20% ethyl acetate in hexane. The title compound was obtained as a white powder.

Step 2: Preparation of 4-aminomethyl-2-methoxypyridine

A solution of 4-cyano-2-methoxypyridine (0.55 g, 4.1 mmol) in ethanol was hydrogenated at 60 psi $H_2$ in the presence of 10% Pd/C (100 mg). After 3.5 h the catalyst was removed by filtration through Super-Cel and the filtrate evaporated to give the title compound as a foam.

Step 3: Preparation of N-(3-methoxy-4-pyridylmethyl)-5-chloro-3-phenylthioindole-2-carboxamide

The title compound was prepared according to the procedure described in example 1, step B, except substituting 4-aminomethyl-2-methoxypyridine for 3-aminomethylpyridine. The dimethylformamide was removed in vacuo and the crude product purified by chromatography on silica gel with 20-40% ethyl acetate in hexane. The title compound was obtained as a white solid, mp 227-228 °C. Anal. Calc. for $C_{22}H_{18}ClN_3O_2S$: C, 62.33; H, 4.28; N, 9.91. Found: C, 62.63; H, 4.21; N, 9.92. NMR (DMSO-$d_6$): δ 12.58 (1H, s), 8.93 (1H), 8.37 (2H, d), 7.56 (1H, d), 7.47 (1H, s), 7.27 (3H, m), 7.18 (2H, m), 7.05 (2H, d), 4.59 (2H, d), 3.30 (3H, s).

**EXAMPLE 23**

**Preparation of N-(3-hydroxymethyl)benzyl-5-chloro-3-phenylthioindole-2-carboxamide**

The title compound was prepared according to the procedure described in example 1, step B, except substituting 3-hydroxymethylbenzylamine for 3-aminomethylpyridine. The dimethylformamide was removed in vacuo and the crude product recrystallized from acetonitrile. The title compound was obtained as a white solid, mp 229-230 °C. Anal. Calc. for $C_{22}H_{17}ClN_2O_2S$: C, 64.61; H, 4.19; N, 6.85. Found: C, 64.20; H, 4.09; N, 6.85. NMR (DMSO-$d_6$): δ 12.69 (1H, s), 10.33 (1H, s), 7.60 (3H, m), 7.49 (1H, s), 7.30 (4H, m), 7.15 (4H, m), 5.22 (1H, t, J=7 Hz), 4.50 (2H, d, J=7 Hz).

**EXAMPLE 24**

**Preparation of N-(3-hydroxybenzyl)-5-chloro-3-phenylthioindole-2-carboxamide**

The title compound was prepared according to the procedure described in example 1, step B, except substituting 3-hydroxybenzylamine for 3-aminomethylpyridine. The dimethylformamide was removed in vacuo and the crude product was chromatographed on silica gel with 10% methanol in chloroform. The title compound was obtained as a white solid, mp 214-216°C. Anal. Calc. for $C_{22}H_{17}ClN_2O_2S \cdot 0.3\ H_2O$; C, 63.77; H, 4.04; N, 6.76. Found: C, 63.92; H, 3.88; N, 6.49. NMR (DMSO-$d_6$): δ 12.55 (1H, s), 9.34 (1H, s), 8.75 (1H, t, J=5 Hz), 7.53 (1H, d, J=8 Hz), 7.45 (1H, s), 7.1-7.65 (4H, m), 7.06 (2H, d, J=7 Hz), 7.01 (1H, t, J=8 Hz), 6.70 (1H, s), 6.62 (2H, m), 4.48 (2H, d, J=5 Hz).

EXAMPLE 25

Preparation of 5-Chloro-3-phenylsulfonylindole-2-carboxamide (Compound 18)

5-Chloro-3-phenylthioindole-2-carboxamide (0.177 g, 0.584 mmol) was dissolved in 25 mL chloroform and cooled to 0°C. 50% By weight meta-chloroperoxybenzoic acid (503 mg, 1.46 mmol) was added and the reaction stirred at 20°C for 6 hours. A 10% aqueous solution of sodium thiosulfate was added and the reaction vigorously stirred for 10 minutes. The layers were separated and the organic phase washed with saturated sodium chloride then dried over magnesium sulfate. The crude product was chromatographed over silica gel eluting with 40% ethyl acetate in hexane. The title compound was obtained as a white powder, mp 255-257°C. NMR (300 MHz, DMSO-d6): δ 13.05 (1H,s), 8.48(1H,s), 8.25(1H,s), 8.03(2H,d,J=8 Hz), 7.95(1H,s), 7.60(4H,m), 7.34(1H,d,J=8 Hz). Anal. Calc. for $C_{15}H_{11}ClN_2O_3S$: C, 53.82; H, 3.31; N, 8.37. Found: C, 53.74; H, 3.29; N, 8.34

EXAMPLE 26

Preparation of 5-Chloro-3-phenylsulfinylindole-2-carboxamide (Compound 17)

A solution of magnesium monoperoxyphthalic acid (85% peracid) (11.8 mg, 0.024 mmol) in methanol (2 mL) was added dropwise to a solution of 5-chloro-3-phenylthioindole-2-carboxamide (14.5 mg, 0.048 mmol) in methanol (2 mL) at 0°C. The reaction was stirred at 20°C for 4 hours. A solution of 10% aqueous sodium thiosulfate was added and the reaction stirred vigorously for 10 minutes. Methanol was removed in vacuo and the residue partitioned between ethyl acetate and water. The ethyl acetate extract was washed with brine and dried over magnesium sulfate. The crude product was purified by column chromatography on silica gel with 30-40% ethyl acetate in hexane. The title compound was obtained as a white solid. NMR (DMSO-$d_6$, 300 MHz) δ 12.53(1H, s), 8.35(1H,br s), 8.08(1H,br s), 7.83(1H,d,J=2 Hz), 7.71(2H,d,J=8 Hz), 7.52(4H,m), 7.30(1H,dd,J=9,2 Hz).

While the foregoing specification teaches the principles of the present invention, with examples provided for the purpose of illustration, it will be understood that the practice of the invention encompasses all of the usual variations, adaptations, and modifications, as come within the scope of the following claims and its equivalents.

## Claims

**1.** A compound of formula A,

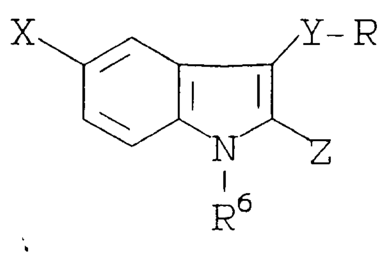

A

wherein

X is -H, -Cl, -F, -Br, $-NO_2$, -CN, or $-OR^2$;

Y is $-S(O)_n-$, $-CR^2R^2-$ or -O-, wherein n is zero, 1 or 2;

R is

1) hydrogen,
2) $-C_{1-5}$alkyl, unsubstituted or substituted with one or more of:
   a) $-C_{1-5}$alkyl,
   b) $-C_{1-5}$alkoxy,
   c) -OH, or
   d) aryl, unsubstituted or substituted with one or more of:
      i) $-C_{1-5}$alkyl,
      ii) $-C_{1-5}$alkoxy,
      iii) -OH, or
      iv) halogen,
3) aryl, unsubstituted or substituted with one or more of:
   a) $-C_{1-5}$alkyl, unsubstituted or substituted with one or more of:
      i) -OH or
      ii) $-C_{1-5}$alkoxy,
   b) $-C_{1-5}$alkoxy,
   c) -OH, or
   d) halogen, or
4) heterocycle, unsubstituted or substituted with one or more of:
   a) $-C_{1-5}$alkyl, unsubstituted or substituted with one or more of:
      i) -OH or
      ii) $-C_{1-5}$alkoxy,
   b) $-C_{1-5}$alkoxy, or
   c) -OH;

Z is

1)

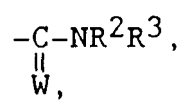

wherein W is O, S, -N-CN or $-N-OR^1$,

2)

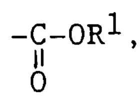

3)

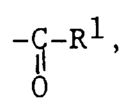

4)

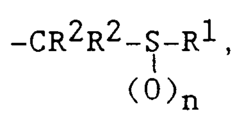

wherein n is defined above,
5) $-CR^2R^2NHR^4$,
6)

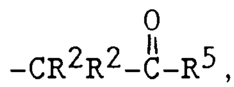

7) $-C_{1-3}$alkyl, unsubstituted or substituted with one or more of:
  a) aryl, unsubstituted or substituted with one or more of:
    i) $-C_{1-5}$alkyl,
    ii) $-C_{1-5}$alkoxy,
    iii) -OH or
    iv) halogen, or
  b) heterocycle, unsubstituted or substituted with one or more of:
    i) $-C_{1-5}$alkyl,
    ii) $-C_{1-5}$alkoxy, or
    iii) -OH, or
8) -CN;

$R^1$ is
1) hydrogen,
2) $-C_{1-5}$alkyl, unsubstituted or substituted with one or more of:
  a) $-C_{1-5}$alkyl,
  b) $-C_{1-5}$alkoxy,
  c) -OH,
  d) aryl, unsubstituted or substituted with one or more of:
    i) $-C_{1-5}$alkyl,
    ii) $-C_{1-5}$alkoxy, or
    iii) -OH, or
  e) heterocycle, unsubstituted or substituted with one or more of:
    i) $-C_{1-5}$alkyl,
    ii) $-C_{1-5}$alkoxy, or
    iii) -OH, or

3) aryl, unsubstituted or substituted with one or more of:
  a) $-C_{1-5}$alkyl, unsubstituted or substituted with one or more of:
    i) -OH or
    ii) $-C_{1-5}$alkoxy,
  b) $-C_{1-5}$alkoxy,
  c) -OH,
  d) halogen,
  e) -CN,
  f) $-NO_2$, or
  g) $-NR^2R^2$; or
4) heterocycle, unsubstituted or substituted with one or more of:
  a) $-C_{1-5}$alkyl, unsubstituted or substituted with one or more of:
    i) -OH or
    ii) $-C_{1-5}$alkoxy,
  b) $-C_{1-5}$alkoxy, or
  c) -OH;

$R^2$ is hydrogen or $C_{1-3}$alkyl;

$R^3$ is
1) $-C_{1-5}$alkyl, unsubstituted or substituted with one or more of:
  a) $-C_{1-5}$alkyl,
  b) $-C_{1-5}$alkoxy, unsubstituted or substituted with -OH,

c) -OH,
d)

$$-O\underset{\underset{O}{\|}}{C}R^7;$$

e) $-COOR^2$
f) aryl, unsubstituted or substituted with one or more of:
i) $-C_{1-5}$alkyl, unsubstituted or substituted with one or more of -OH,
ii) $-C_{1-5}$alkoxy,
iii) -OH, or
iv) halogen,
g) heterocycle, unsubstituted or substituted with one or more of:
i) $-C_{1-5}$alkyl,
ii) $-C_{1-5}$alkoxy,
iii) -OH,
iv) $C_{1-3}$alkyl-$NR^2R^2$
h) $-NR^2R^2$,
i) $-C_{3-6}$cycloalkyl,
2) aryl, unsubstituted or substituted with one or more of:
a) $-C_{1-5}$alkyl,
b) $-C_{1-5}$alkoxy,
c) -OH,
d) halogen,
3) heterocycle, unsubstituted or substituted with one or more of:
i) $-C_{1-5}$alkyl,
ii) $-C_{1-5}$alkoxy,
iii) -OH,

4) $-C_{1-5}$alkoxy,
5) -OH,
6) $-C_{3-6}$cycloalkyl, or
7) hydrogen;

$R^4$ is
1) $R^1$ or
2)

$$-\underset{\underset{O}{\|}}{C}R^1;$$

$R^5$ is
1) $R^1$,
2) $-C_{1-5}$alkoxy,
3) $-NHR^1$, or
4) heterocycle, unsubstituted or substituted with one or more of:
i) $-C_{1-5}$alkyl,
ii) $-C_{1-5}$alkoxy,
iii) -OH;

$R^6$ is
1) hydrogen,
2)

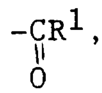

or

3)

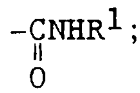

and

$R^7$ is

1) aryl, unsubstituted or substituted with one or more of -Cl, -Br, -OH, $-OCH_3$, or -CN, or

2) $-C_{1-5}$alkyl, unsubstituted or substituted with one or more of -OH or $-NR^2R^2$, with the proviso that when X is -H, Y is -S, R is unsubstituted phenyl and $R^6$ is -H, Z is not

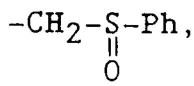 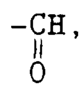

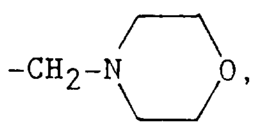 or 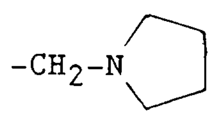 ;

or a pharmaceutically acceptable salt or ester thereof.

**2.** A compound according to Claim 1 wherein:

X is -H, -Cl or -F;

Y is $-S(O)_n-$ or $-CH_2-$;

R is -Ph, or -tolyl;

$R^6$ is -H; and

Z is

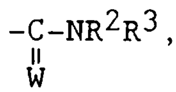 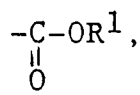 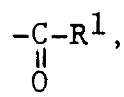

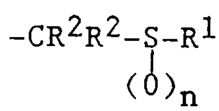

or

$-C_{1-3}$alkyl substituted with heterocycle.

**3.** A compound according to Claim 2 wherein:

X is -H or -Cl;

Y is $-S(O)_n-$;

R is -Ph, or -tolyl;

$R^6$ is -H; and

Z is

$$-\underset{\underset{W}{\|}}{C}-NR^2R^3,$$

wherein $R^2$ is -H and W is -O or -S, or

$$-CR^2R^2-\overset{\overset{O}{\|}}{S}-aryl,$$

wherein the aryl group is unsubstituted or substituted with one or more of $C_{1-5}$alkyl.

**4.** A compound according to Claim 3 wherein:

X is -Cl;
Y is $-S(O)_n-$;
R is -Ph;
$R^6$ is -H;
Z is

$$-\underset{\underset{W}{\|}}{C}-NR^2R^3,$$

wherein $R^2$ is -H, and W is -O or -S; and

$R^3$ is

1) $-C_{1-5}$alkyl, unsubstituted or substituted with one or more of
   a) $-C_{1-5}$alkoxy, unsubstituted or substituted with -OH,
   b) -OH,
   c)

$$-OC\underset{\underset{O}{\|}}{}R^7,$$

   d) aryl, unsubstituted or substituted with one or more of:
      i) $-C_{1-5}$alkyl, unsubstituted or substituted with one or more of -OH,
      ii) $-C_{1-5}$alkoxy,
      iii) -OH,
   e) heterocycle, unsubstituted or substituted with one or more of:
      i) $-C_{1-5}$alkyl,
      ii) $-C_{1-5}$alkoxy, or
      iii) -OH,

2) heterocycle, unsubstituted or substituted with one or more of:
   i) $-C_{1-5}$alkyl,
   ii) $-C_{1-5}$alkoxy, or
   iii) -OH, or
3) hydrogen.

**5.** The compound,

N-ethyl-5-chloro-3-phenylthioindole-2-carboxamide,
N-2-hydroxyethyl-5-chloro-3-phenylthioindole-2-carboxamide,
N-2-methoxyethyl-5-chloro-3-phenylthioindole-2-carboxamide,
N-3-methoxybenzyl-5-chloro-3-phenylthioindole-2-carboxamide,
N-4-pyridylmethyl-5-chloro-3-phenylthioindole-2-carboxamide,
N-3-pyridylmethyl-5-chloro-3-phenylthioindole-2-carboxamide,

N-2-furanylmethyl-5-chloro-3-phenylthioindole-2-carboxamide,
N-3-pyridyl-5-chloro-3-phenylthioindole-2-carboxamide,
N-[1-(2(R)-hydroxypropyl)]-5-chloro-3-phenylthioindole-2-carboxamide,
N-(2-pyridyl)methyl-5-chloro-3-phenylthioindole-2-carboxamide,
N-(3-methoxy-4-pyridyl)methyl-5-chloro-3-phenylthioindole-2-carboxamide,
N-(3-hydroxymethyl)benzyl-5-chloro-3-phenylthioindole-2-carboxamide,
5-chloro-3-phenylthioindole-2-carboxamide,
N-(3-hydroxybenzyl)-5-chloro-3-phenylthioindole-2-carboxamide,
N-2-furanylmethyl-5-chloro-3-phenylthioindole-2-thiocarboxamide,
5-chloro-3-phenylthioindole-2-thiocarboxamide,
5-chloro-3-phenylsulfinylindole-2-carboxamide, or
5-chloro-3-phenylsulfonylindole-2-carboxamide,
or a pharmaceutically acceptable salt or ester thereof.

**6.** The compound,
N-ethyl-5-chloro-3-phenylthioindole-2-carboxamide, N-2-hydroxyethyl-5-chloro-3-phenylthioindole-2-carboxamide,
N-2-methoxyethyl-5-chloro-3-phenylthioindole-2-carboxamide,
N-3-methoxybenzyl-5-chloro-3-phenylthioindole-2-carboxamide,
N-4-pyridylmethyl-5-chloro-3-phenylthioindole-2-carboxamide,
N-3-pyridylmethyl-5-chloro-3-phenylthioindole-2-carboxamide,
N-2-furanylmethyl-5-chloro-3-phenylthioindole-2-carboxamide, or
N-3-pyridyl-5-chloro-3-phenylthioindole-2-carboxamide,
or a pharmaceutically acceptable salt or ester thereof.

**7.** The compound,
N-[1-(2(R)-hydroxypropyl)]5-chloro-3-phenylthioindole-2-carboxamide,
N-(2-pyridyl)methyl-5-chloro-3-phenylthioindole-2-carboxamide,
N-(3-methoxy-4-pyridyl)methyl-5-chloro-3-phenylthioindole-2-carboxamide,
N-(3-hydroxymethyl)benzyl-5-chloro-3-phenylthioindole-2-carboxamide,
5-chloro-3-phenylthioindole-2-carboxamide,
N-(3-hydroxybenzyl)-5-chloro-3-phenylthioindole-2-carboxamide,
N-2-furanylmethyl-5-chloro-3-phenylthioindole-2-thiocarboxamide,or
5-chloro-3-phenylthioindole-2-thiocarboxamide,
or a pharmaceutically acceptable salt or ester thereof.

**8.** The compound
5-chloro-3-phenylsulfinylindole-2-carboxamide, or
5-chloro-3-phenylsulfonylindole-2-carboxamide,
or a pharmaceutically acceptable salt or ester thereof.

**9.** The use of a compound as claimed in any of Claims 1-8 for the manufacture of a medicament for inhibiting HIV reverse transcriptase.

**10.** The use of a compound as claimed in any of Claims 1-8 for the manufacture of a medicament for the treatment or prevention of infection of HIV, or the treatment of AIDS or ARC.

**11.** A pharmaceutical composition comprising a compound as claimed in any of Claims 1-8 and a pharmaceutically acceptable carrier or excipient.

European Patent Office

# EUROPEAN SEARCH REPORT

Application Number
EP 95 20 1691

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | CHEMICAL ABSTRACTS, vol. 72, no. 3, 19 January 1970 Columbus, Ohio, US; abstract no. 12471d, 'synthesis of 3-benzylindole deriv.' page 313; * abstract * & KHIM.-FARM ZH, vol. 3, no. 7, 1969 pages 10-15, V I SHVEDOV --- | 1,2 | C07D209/42<br>C07D209/30<br>C07D401/12<br>C07D405/12<br>C07D403/12<br>C07D401/06<br>C07D405/06<br>C07D413/06<br>A61K31/40<br>A61K31/44<br>A61K31/41 |
| X | CHEMICAL ABSTRACTS, vol. 85, no. 5, 2 August 1976 Columbus, Ohio, US; abstract no. 32754v, 'convenient preparation of 2,3-dialkylindoles' page 374; * abstract * & ORG. PREP. PROCED. INT., vol. 8, no. 1, 1976 pages 41-44, D VINOD --- | 1 | TECHNICAL FIELDS SEARCHED (Int.Cl.5)<br>C07D<br>A61K |
| X | CHEMICAL ABSTRACTS, vol. 87, no. 17, 24 October 1977 Columbus, Ohio, US; abstract no. 135057r, '3-indolyl thio ethers' page 705; * abstract * & JP-A-07 739 761 (SANKYO CO LTD) 28 March 1977 --- | 1,11 | |
| | -/-- | | |

The present search report has been drawn up for all claims

| Place of search | Date of completion of the search | Examiner |
|---|---|---|
| MUNICH | 28 July 1995 | Scruton-Evans, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 95 20 1691

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | CHEMICAL ABSTRACTS, vol. 90, no. 1, 1 January 1979 Columbus, Ohio, US; abstract no. 6272v, 'metal reagents in organic reactions:part II' page 575; * abstract * & INDIAN J CHEM SECT B, vol. 16b, no. 5, 1978 pages 422-424, BANERJI A<br>--- | 1 | |
| X | CHEMICAL ABSTRACTS, vol. 106, no. 7, 16 February 1987 Columbus, Ohio, US; abstract no. 49926c, 'photochemical conversion of 3-azido-2-vinylthiophenes into thienopyrroles and of 2-azidostyrenes into indoles' page 616; * abstract * & J CHEM SOC PERKIN TRANS 1, vol. 3, 1986 pages 501-6, GAIRNS R<br>--- | 1,2 | TECHNICAL FIELDS SEARCHED (Int.Cl.5) |
| X | CHEMICAL ABSTRACTS, vol. 109, no. 17, 24 October 1988 Columbus, Ohio, US; abstract no. 149279z, 'a new synthesis of 3-arylthioindoles' page 710; * abstract * & SYNTHESIS, vol. 6, 1988 pages 480-1, ATKINSON J G<br>---<br>-/-- | 1,2 | |

The present search report has been drawn up for all claims

| Place of search | Date of completion of the search | Examiner |
|---|---|---|
| MUNICH | 28 July 1995 | Scruton-Evans, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number
EP 95 20 1691

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | CHEMICAL ABSTRACTS, vol. 106, no. 15, 13 April 1987 Columbus, Ohio, US; abstract no. 119608b, 'reactions of organic anions, part 132' page 618; * abstract * & SYNTHESIS, vol. 8, 1986 pages 651-3, K WOJCIECHOWSKI --- | 1 | |
| X | REMERS AND SPANDE 'indoles part three (the chemistry of heterocyclic compounds, vol 25' 1979 , JOHN WILEY AND SONS , USA pages 306,309,310,314,315,461,462,476,477 --- | 1 | |
| X | DE-A-19 35 671 (SUMITOMO CHEMICAL COMPANY LTD) 5 March 1970 * claims 1-3 * --- | 1,2 | TECHNICAL FIELDS SEARCHED (Int.Cl.5) |
| Y | WO-A-91 09849 (UPJOHN CO) 11 July 1991 * claims 1,10-12 * --- | 1-11 | |
| A | CHEMICAL ABSTRACTS, vol. 104, no. 1, 7 July 1986 Columbus, Ohio, US; abstract no. 61594j, 'Reversal of feline retroviral suppression by indomethacin' page 26; * abstract * & LEUK RES, vol. 9, no. 12, 1985 pages 1451-6, M LEWIS --- | 1-11 | |
| | -/-- | | |

The present search report has been drawn up for all claims

| Place of search | Date of completion of the search | Examiner |
|---|---|---|
| MUNICH | 28 July 1995 | Scruton-Evans, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number
EP 95 20 1691

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| Y | WO-A-91 05761 (UNIROYAL CHEM LTD) 2 May 1991<br>whole document, especially definition iii for R, with rz as definition d), and RA as defintion 3<br>----- | 1-11 | |

TECHNICAL FIELDS SEARCHED (Int.Cl.5)

The present search report has been drawn up for all claims

| Place of search | Date of completion of the search | Examiner |
|---|---|---|
| MUNICH | 28 July 1995 | Scruton-Evans, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)